(19) Europäisches Patentamt
European Patent Office
Office européen des brevets

(11) Publication number: **0 375 183**
**A1**

(12) **EUROPEAN PATENT APPLICATION**

(21) Application number: **89312467.7**

(22) Date of filing: **30.11.89**

(51) Int. Cl.$^5$: **C07H 19/04, C07H 19/10, C07H 19/20, A61K 31/70**

(30) Priority: **05.12.88 US 279745**

(43) Date of publication of application: **27.06.90 Bulletin 90/26**

(84) Designated Contracting States: **GR**

(71) Applicant: **SCHERING CORPORATION**
**2000 Galloping Hill Road**
**Kenilworth New Jersey 07033(US)**

(72) Inventor: **Saksena, Anil K.**
**53 Beverley Road**
**Upper Montclair New Jersey 07043(US)**
Inventor: **Girijavallabhan, Viyyoor M.**
**10 Maplewood Avenue**
**Parsippany New Jersey 07054(US)**
Inventor: **Ganguly, Ashit K.**
**96 Cooper Avenue**
**Upper Montclair New Jersey 07043(US)**

(74) Representative: **Ritter, Stephen David et al**
**Mathys & Squire 10 Fleet Street**
**London EC4Y 1AY(GB)**

(54) **Antiviral dimers and trimers.**

(57) Phosphate and phosphonate dimers and trimers and their use as anti-viral agents are disclosed together with a process of their preparation.

EP 0 375 183 A1

## ANTIVIRAL DIMERS AND TRIMERS

The present invention relates to novel phosphate and phosphonate dimers and trimers useful as antiviral agents.

Cyclopentyl purine derivatives are compounds disclosed as possessing antiviral activity. European Patent Application 236935 teaches certain cyclopentyl derivatives as having antiviral activity against viruses such as DNA viruses, herpes virus group (e.g. herpes simplex virus type I or II, cytomegalovirus, Epstein-Barr virus), Hepatitis B virus; or RNA viruses such as human immunodeficiency virus (HIV) which is a pathogen of acquired immunodeficiency syndrome (AIDS), vesicular stomatitis virus, feline leukemia virus and equine infectious anemic virus.

European Patent Application 219838 discloses analogues of cyclopentyl purine derivatives.

A review paper by Victor E. Marquez and Mu-Ill Lim (1986) Medicinal Research Reviews, Vol. 6, No. 1, pages 1-40 by John Wiley & Sims, Inc. teaches numerous cyclopentyl derivatives as well as methods for their preparation.

## SUMMARY OF THE INVENTION

It has now surprisingly been found that advantageous anti-viral activity is provided by (1) a compound of the formula (I)

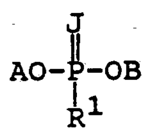

(I)

or a pharmaceutically acceptable salt or solvate thereof, wherein:

J represents an oxygen or sulfur atom,

A represents

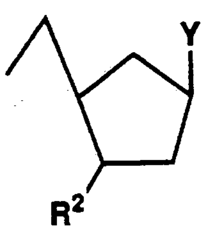 , 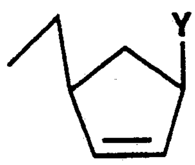 , 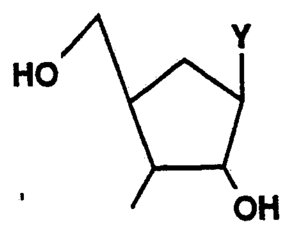 or 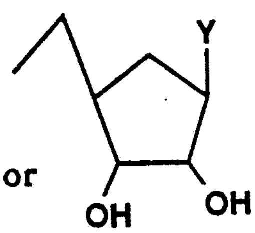 ;

B represents

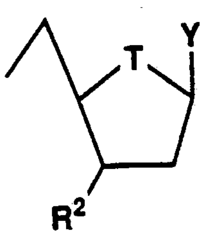 , 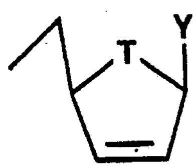 , 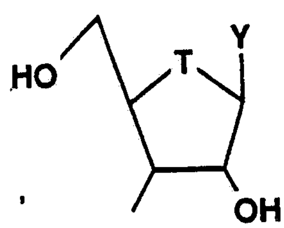 or 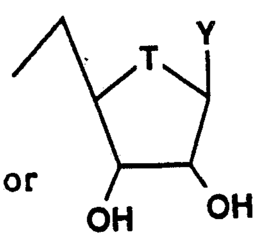 ;

$R^1$ represents hydroxy (OH), alkyl, alkoxy, hydroxyalkyl, alkoxyalkyl, alkoxyalkoxy, acyl, phenoxy, substituted phenoxy, or OB where B is defined as above and each B may be the same or different;

each Y independently represents

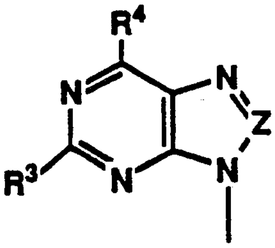 , 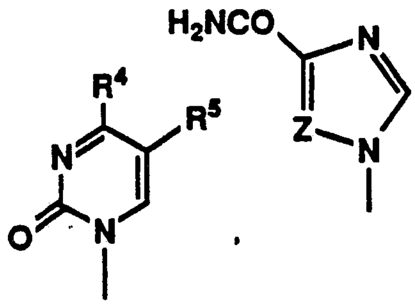 , or 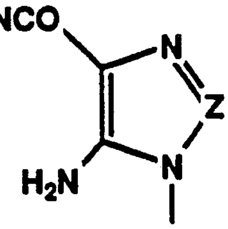 ;

each $R^2$ independently represents H, F, (F,F), OH, $NO_2$, azido, alkoxy, amino, alkylamino, dialkylamino or amide;
each T independently represents O or $CH_2$;
$R^3$ and $R^4$ can be the same or different and each is independently selected from H, halo, OH, SH, alkoxy, alkylthio, amino, alkylamino, dialkylamino or amide;
each $R^5$ independently represents H, halo, $-CF_3$, vinyl, halovinyl, alkyl, hydroxyalkyl, alkoxyalkyl, -COOH, -COOalkyl or acyl; and
each Z independently represents N or CH. Preferably, J is O, $R^1$ is OH and B represents

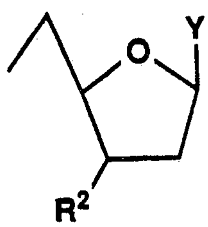 , 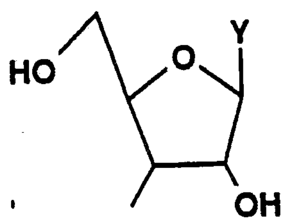 or 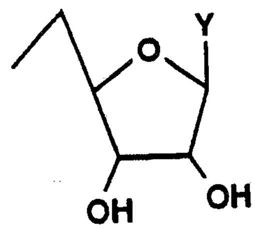 ;

wherein $R^2$ and Y are as defined above. In one embodiment J is O, $R^1$ is OH and B represents the same values as for A as defined above.

Each Y group is preferably independently selected from

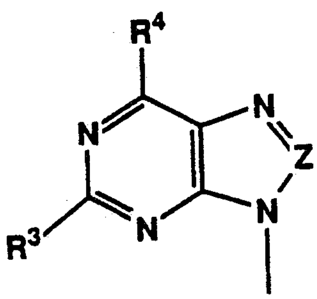 , 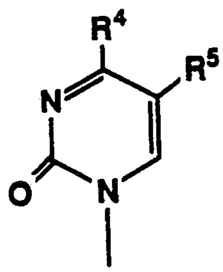 or 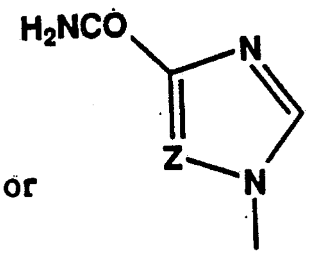 ;

wherein $R^3$, $R^4$ and Z are as defined above, and each $R^5$ represents halo, alkyl, $-CF_3$, vinyl, halovinyl, $-CH_2OH$, $-CH_2Oalkyl$, -COOH or -COOalkyl.

Preferred embodiments of the compounds of the invention are represented by formulas Ia-In below:

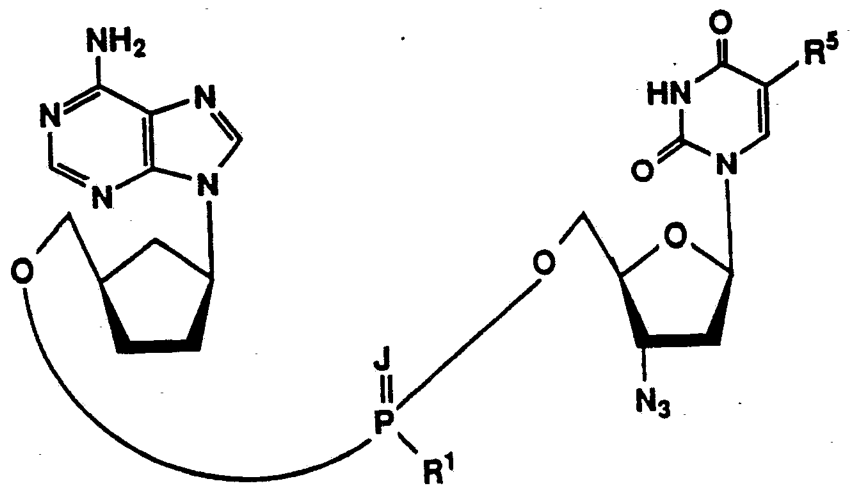

Ia

wherein J is oxygen (O) or sulfur (S), $R^1$ is OH, $OCH_3$, $CH_3$ or OB where B is as defined above, and $R^5$ is H, $CH_3$ or halo;

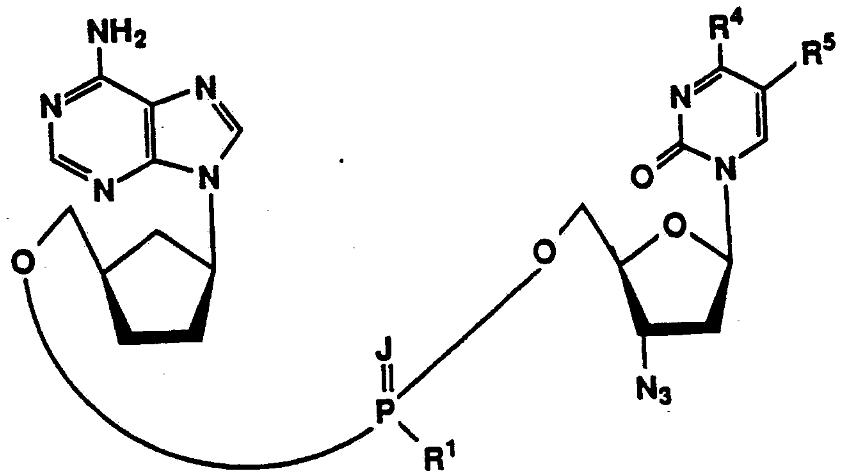

Ib

wherein J is O or S, $R^1$ is OH, $OCH_3$, or $CH_3$, $R^4$ is OH or $NH_2$, and $R^5$ is H, $CH_3$ or halo;

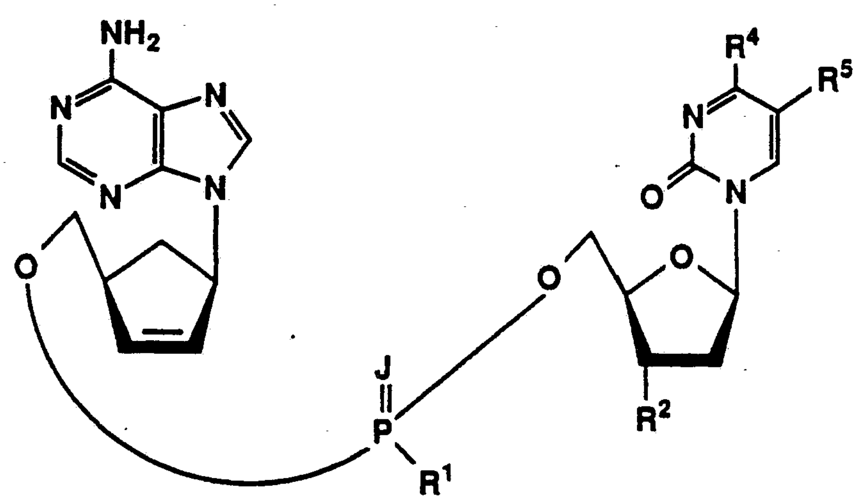

Ic

wherein J is O or S, $R^1$ is OH, $OCH_3$, $CH_3$ or OB where B is as defined hereinbefore, $R^2$ is H or $N_3$, $R^4$ is OH or $NH_2$, and $R^5$ is H, $CH_3$ or halo;

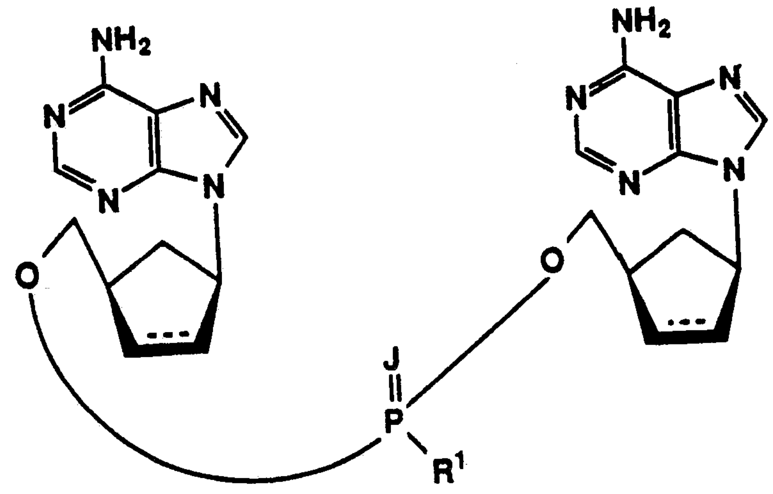

Id

wherein J is O or S, $R^1$ is OH, $OCH_3$, or $CH_3$ or OB where B is as defined hereinbefore, and the dotted lines indicate optional double bonds;

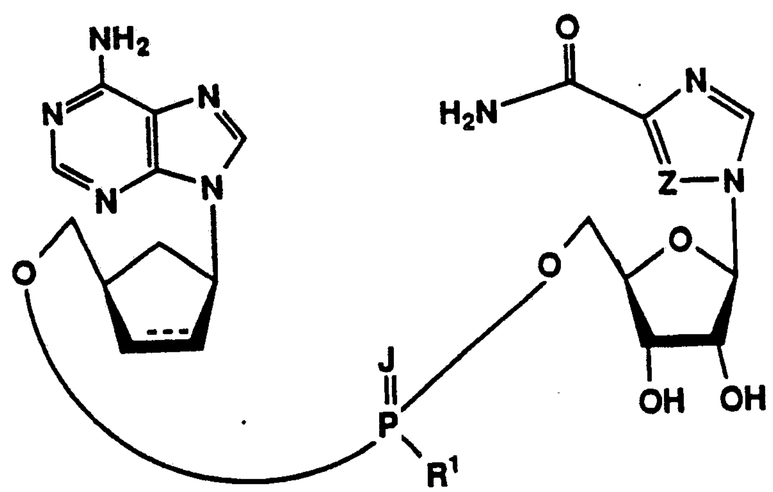

Ie

wherein J is O or S, $R^1$ is OH, $OCH_3$, $CH_3$ or OB and B is as defined above, Z is N or CH, and the dotted line indicates an optional double bond;

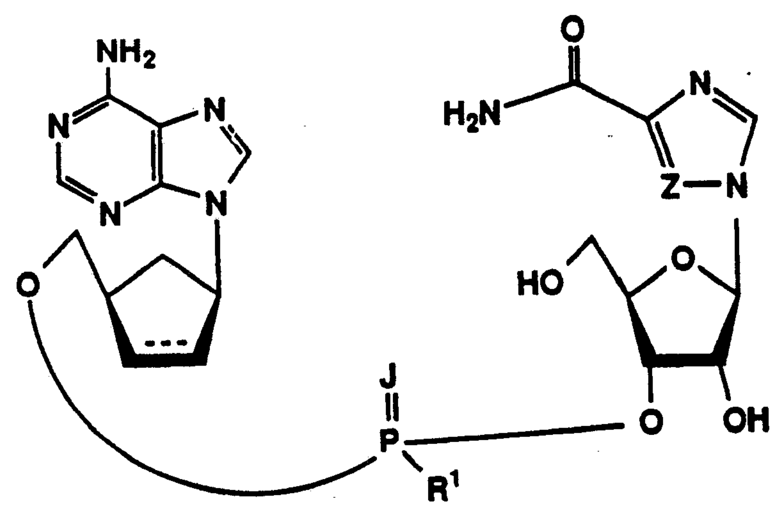

If

wherein J is O or S, $R^1$ is OH, $OCH_3$, $CH_3$ or OB and B is as defined above, Z is N or CH, and the dotted line indicated an optional double bond;

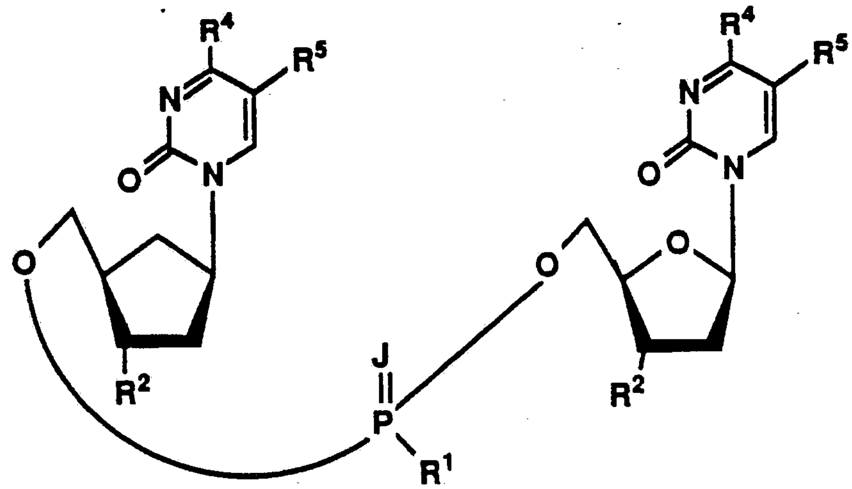

Ig

wherein J is O or S, $R^1$ is OH, $OCH_3$, $CH_3$ or OB and B is as defined hereinbefore, $R^2$ is H or $N_3$, each $R^4$ is independently OH or $NH_2$, and each $R^5$ is independently H, $CH_3$ or halo;

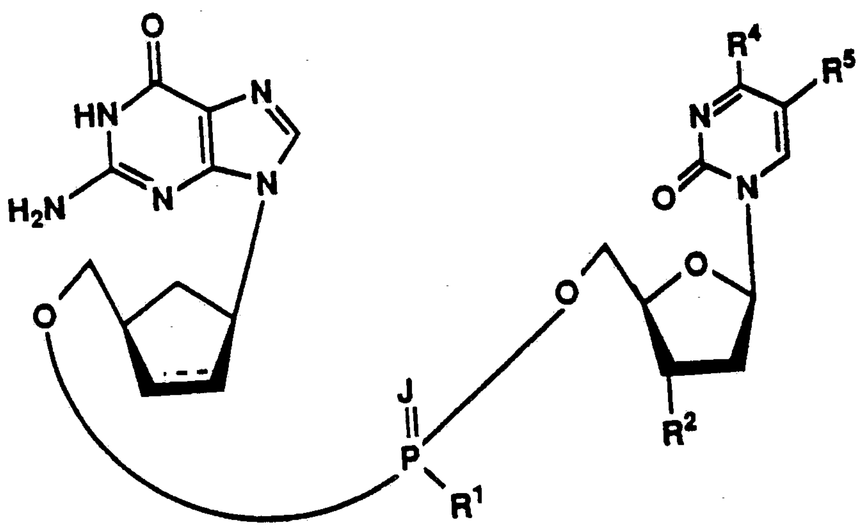

Ih

wherein J is O or S, $R^1$ is OH, $OCH_3$, $CH_3$ or OB and B is as defined hereinbefore, $R^2$ is H or $N_3$, $R^4$ is OH or $NH_2$, $R^5$ is H, $CH_3$ or halo, and the dotted line indicates an optional double bond;

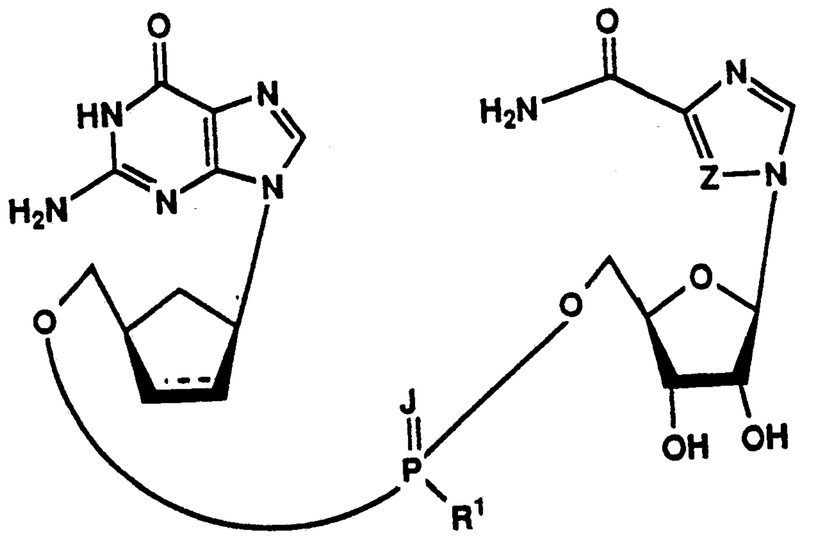

Ii

wherein J is O or S, $R^1$ is OH, $OCH_3$, $CH_3$ or OB and B is as defined hereinbefore, Z is N or CH, and the dotted line indicates an optional double bond;

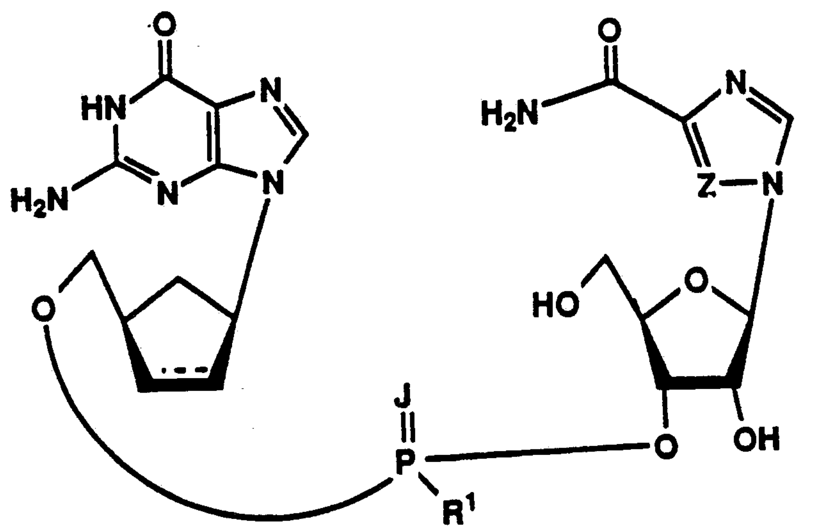

Ij

wherein J is O or S, $R^1$ is OH, $OCH_3$, $CH_3$ or OB whereB is as defined hereinbefore, Z is N or CH, and the dotted line indicates an optional double bond;

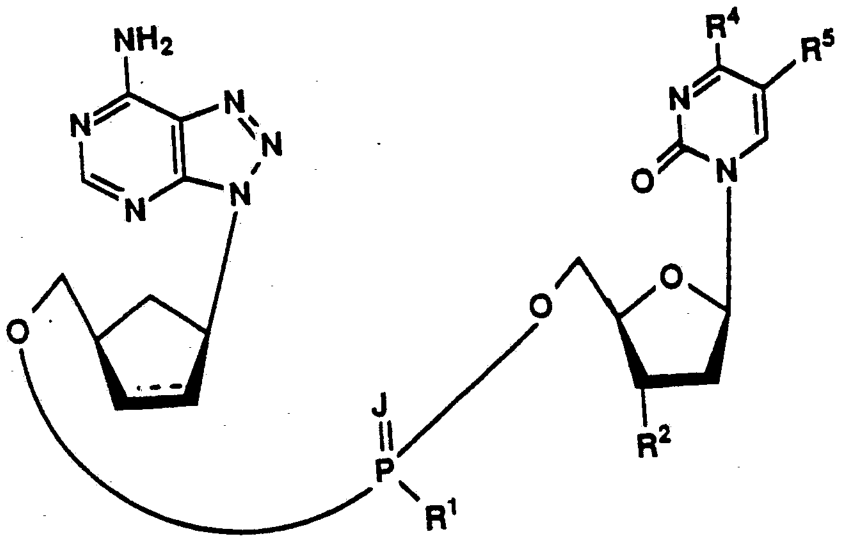

Ik

wherein J is O or S, $R^1$ is OH, $OCH_3$, $CH_3$ or OB where B is as defined hereinbefore, $R^2$ is H or $N_3$, $R^4$ is OH or $NH_2$, $R^5$ is H, $CH_3$ or halo and the dotted line indicates an optional double bond;

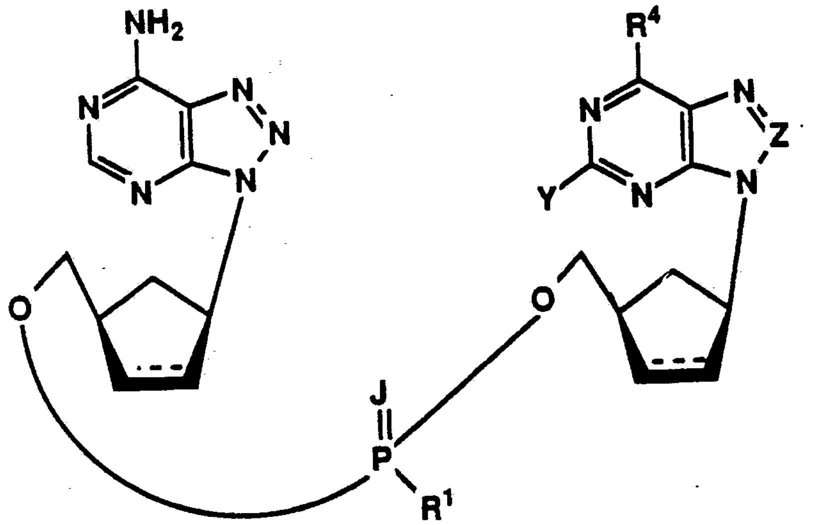

Il

wherein J is O or S, $R^1$ is OH, $OCH_3$, $CH_3$ or OB where B is as defined hereinbefore, $R^3$ is H or $NH_2$, $R^4$ is OH or $NH_2$, Z is N or CH, and the dotted lines indicate optional double bond;

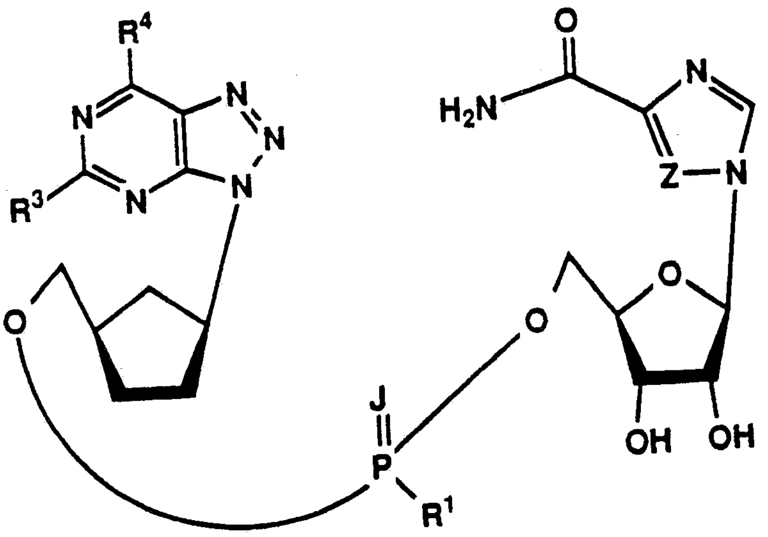

Im

wherein J is O or S, $R^1$ is OH, $OCH_3$, $CH_3$ or OB where B is as defined hereinbefore, $R^3$ is H or $NH_2$, $R^4$ is OH or $NH_2$ and Z is N or CH; or

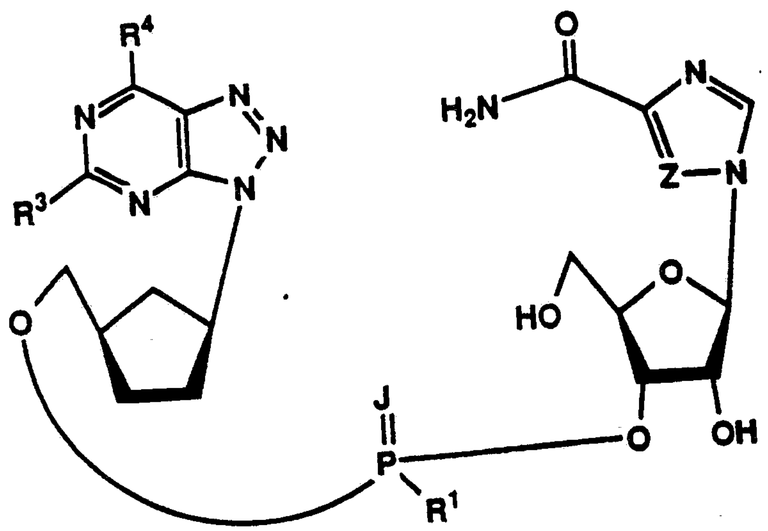

In

wherein J is O or S, $R^1$ is OH, $OCH_3$, $CH_3$ or OB where B is as defined hereinbefore, $R^3$ is H or $NH_2$, $R^4$ is OH or $NH_2$, and Z is N or CH.

Most preferably the compound of formula I is 5′-O-[[[3-(6-amino-9H-purin-9-yl)-cyclopentyl]methoxy]hydroxyphosphinyl]-3′-azido-3′-deoxy-thymidine.

Other aspects of the invention include (2) a pharmaceutical composition comprising a pharmaceutically effective amount of a compound of formula I as defined above in combination with a pharmaceutically acceptable carrier and (3) a method of treating susceptible viral infections in a mammal by administering an anti-viral effective amount of a compound of formula I.

The compounds of the present invention are useful as antiviral agents, particularly against viruses such as HIV.

## DETAILED DESCRIPTION OF THE INVENTION

The following terms have the meanings listed below, unless otherwise indicated herein:

alkyl (including each alkyl portion of alkoxy, hydroxyalkyl, alkoxyalkyl, hydroxyalkoxy, alkoxyalkoxy, alkylamino, dialkylamino, -COOalkyl or alkylthio) -represents an unsaturated, straight or branched-chain

hydrocarbon chain having from 1 to 12 carbon atoms, preferably from 1 to 6 carbon atoms;
alkoxy - represents an alkyl group containing from 1 to 12 carbon atoms, preferably from 1 to 6 carbon atoms, the alkyl group being covalently bonded to an adjacent structural element through an oxygen atom, for example, methoxy (i.e. $-OCH_3$), ethoxy, propoxy, hexoxy and the like;
substituted phenyl (including the substituted phenyl of substituted phenyloxy) - represents a phenyl group on which 1 to 5 of the hydrogens are replaced by a substituent Y, each Y being independently selected from halo, alkyl, alkoxy, alkylthio, -OH, hydroxymethyl, $NO_2$, $-CF_3$, -CN, cycloalkyl, alkynyloxy, alkenyloxy, $-S(O)_p-R^6$ (wherein $R^6$ is alkyl and p is 0, 1 or 2), $-CO-R^7$ (wherein $R^7$ represents alkyl, -OH, $-NH_2$, $-NHR^6$, $N(R^6)_2$ or $-OR^6$ in which $R^6$ is as defined above), $-O-D-COR^7$ (wherein D represents a covalent bond and or a straight or branched alkylene group having from 1 to 4 carbon atoms and $R^7$ is as defined above), $-NH_2$, $-NHR^6$, $-N(R^6)_2$ (wherein $R^6$ is as defined above), -NHCOH, and the like;
alkylene - represents the straight or branched divalent group $(-CH_2-)_t$ wherein t is 1, 2, 3 or 4;
alkenyloxy - represents straight and branched hydrocarbon chains having at least one carbon to carbon double bond and, unless otherwise specified, contains from 3 to 6 carbon atoms, the alkenyl group thereof being bonded to an adjacent structural element through an oxygen atom;
hydroxyalkyl - represents an alkyl group of 1 to 12 carbon atoms, preferably from 1 to 6 carbon atoms in which one or more of the hydrogens is replaced by a hydroxy moiety, for example, hydroxymethyl (i.e. $-CH_2OH$), hydroxyethyl, 1-hydroxypropyl, 2-hydroxypropyl, 3-hydroxybutyl, 4-hydroxyhexyl and the like;
hydroxyalkoxy - represents an alkoxy group of 1 to 12 carbon atoms, preferably from 1 to 6 carbon atoms, in which one or more of the hydrogens is replaced by a hydroxy group;
alkoxyalkyl - represents an alkyl group of 1 to 12 carbon atoms, preferably from 1 to 6 carbon atoms, in which one or more of the hydrogens is replaced by an alkoxy group of 1 to 12 carbon atoms, preferably 1 to 6 carbon atoms;
alkoxyalkoxy - represents an alkoxy group of 1 to 12 carbon atoms, preferably from 1 to 6 carbon atoms, in which one or more of the hydrogens is replaced by an alkoxy group of 1 to 12 carbon atoms, preferably 1 to 6 carbon atoms;
alkynyloxy - represents straight and branched hydrocarbon chains having at least one carbon to carbon triple bond (i.e. -C≡C-) and, unless otherwise specified, contains from 3 to 6 carbon atoms, the alkynyl group thereof being bonded to an adjacent structural element through an oxygen atom;
cycloalkyl - represents saturated carbocyclic rings having from 3 to 7 carbon atoms;
acyl (including the acyl portion of amide)

- represents an alkyl- $\overset{\overset{O}{||}}{C}$ -, benzoyl, or substituted phenyl- $\overset{\overset{O}{||}}{C}$ - group;

amide - represents the $H_2N-\overset{\overset{O}{||}}{C}-$ group in which one or both of the hydrogen atoms can be substituted ty alkyl or phenyl;
azido - represents the group $-N_3$;
amino - represents the group $-NH_2$;
alkylthio - represents the group alkyl-S-;
alkylamino - represents the group alkyl-HN-;
dialkylamino - represents the group $(alkyl)_2N-$,
wherein the number of carbon atoms in each alkyl group can be the same or different;
vinyl - represents the univalent ethenyl group, $CH_2=CH-$;
halovinyl - represents the vinyl group, one of whose hydrogens is substituted with a halogen atom, e.g $CH_2=CHalo-$ where Hal or halo is chloro, bromo, fluoro or iodo; and
(F,F) - represents two fluorine atoms attached to a single carbon atom i.e.

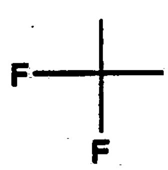 .

As noted hereinabove, the compounds of the invention may include 1 to 5 "Y" substituents on the phenyl rings when such rings are present. In compounds where there is more than one such Y substituent, they may be the same or different. Thus, compounds having combinations of different Y substituents are

contemplated within the scope of the invention. Examples of suitable Y substituents include hydroxy, methyl, chloro, bromo, fluoro, methoxy, cyclohexyl, allyloxy, 2-propynyloxy, methylthio, methylsulfonyl, carboxy, acetoxy, N-methylaminocarbonyl, and the like.

The compounds of the invention can exist in solvated as well as unsolvated forms, including hydrated forms, e.g., hemihydrate. In general, solvated forms can be prepared by the reaction between the compounds of the present invention and a pharmaceutically acceptable solvent such as water, ethanol and the like.

Certain compounds of the invention can exist in isomeric forms. The invention contemplates all such isomers both in pure form and in admixture, including racemic mixtures. The cyclopentyl portion of the compound of formula (I) has the same relative and absolute configuration as conventional nucleosides. Furthermore, when $R^1$ is alkyl, two isomeric forms can exist with the phosphonate at the phosphorus atom.

Certain compounds of the invention can also form pharmaceutically acceptable salts with organic and inorganic acids, e.g. acids which will not hydrolyze the phosphate linkage. Examples of suitable acids for salt formation are acetic, citric, oxalic, malonic, salicylic, malic, fumaric, succinic, ascorbic, lactobionic, maleic, and other mineral and carboxylic acids well known to those in the art. The salts are prepared by contacting the free base form with a sufficient amount of the desired acid to produce a salt in the conventional manner. The free base forms can be regenerated by treating the salt with a suitable dilute aqueous base solution such as dilute aqueous sodium hydroxide, potassium carbonate, ammonia or sodium bicarbonate. The free base forms differ from their respective salt forms somewhat in certain physical properties, such as solubility in polar solvents, but the salts are otherwise equivalent to their respective free base forms for purposes of the invention.

Also, some compounds of this invention are acidic, e.g., when $R^1$ is OH or $R^5$ is COOH, and can form salts with inorganic and organic bases, such as alkali metal hydroxides, amines, and the like.

The phosphate ester compounds of formula I wherein J is oxygen and $R^1$ represents alkoxy, alkoxyalkoxy, phenoxy, substituted phenoxy or OB can be prepared in accordance with the teachings of F. Ramirez and J.F. Marecek, Synthesis, pp 449 (1985) and J.H. Van Broom et al., Nucleic Acids Res., 14, pp 7391 (1986) whose preparative teachings are incorporated herein by reference.

The preparation of the phosphate esters can be schematically illustrated as follows:

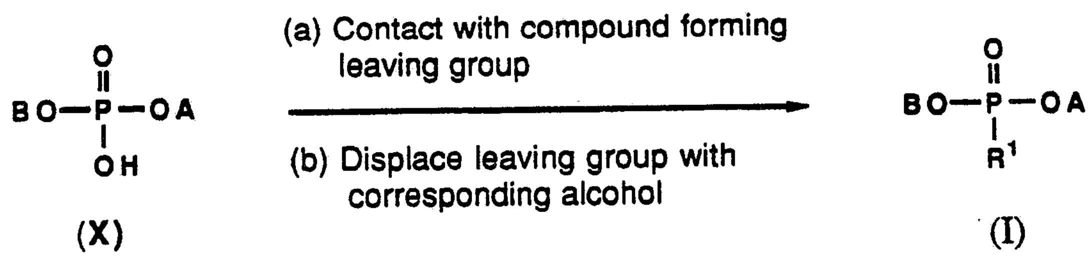


(X) (I)

wherein A and B are as defined hereinbefore and the alcohol is capable of forming the corresponding alkoxy, alkoxyalkoxy, phenoxy, substituted phenyl or OB moiety in compound (I).

Suitable reagents which can form a leaving group (L) on compound of formula (X) include

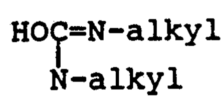

wherein each alkyl is the same or different and represents an alkyl group of 1 to 6 carbon atoms, preferably 6; substituted phenyl-$SO_2OH$ wherein the phenyl is substituted with one or more alkyl of 1 to 6 carbon atoms in the alkyl portion, preferably 2,4,6-trimethylphenyl- or 2,4,6-triisopropylphenyl-;

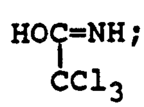

triazoles such as 1-oxybenzotriazole or 1H-1,2,4-triazole; or HN-$(alkyl)_2$ wherein each alkyl can be the same or different and each alkyl is from 1 to 6 carbon atoms, preferably each alkyl is isopropyl.

In step (a), compound of formula (X) is contacted with a reagent which can form a leaving group, L, as

defined hereinbefore in amounts and under conditions effective to allow the leaving group to replace the hydroxyl proton. Generally equimolar or excess amounts of the compound forming the leaving group to compound (X) can be used, at any suitable temperature preferably in the range from about 0 to 30° C, under ambient pressures in any suitable solvent such as methylene chloride ($CH_2Cl_2$), acetonitrile, tetrahydrofuran, pyridine, diethylether, dimethylsulfoxide (DMSO) or mixtures thereof.

In step (b) the product from step (a) can be further contacted with an alcohol, ROH of formula (XX) wherein RO is the same as $R^1$ when $R^1$ is the corresponding alkoxy, alkoxyalkoxy, phenoxy, substituted phenoxy, or OB moiety under conditions effective to displace the leaving group to yield the desired compound (I). Generally, equimolar or excess amounts of the alcohol can be employed, at temperatures and solvents employed in step (a) above.

After the reaction is completed, the desired compound of formula (I) can be recovered by conventional separatory and recovery methods such as phase separation, distillation or evaporation of any solvents present, crystallization, chromatography, filtration and the like.

The preparation of the phosphonate esters of compound (I) wherein J is oxygen and $R^1$ is alkyl, hydroxyalkyl, alkoxyalkyl or acyl can be prepared in accordance with the teachings of M.H. Carruthers, Science, 230, pp. 281 (1985) and W.J. Stec. et al., Tetrahedron Letters, 28, pp. 5535 (1987), whose preparative teachings are incorporated herein by reference. The general preparation of the phosphonate esters (I) is schematically illustrated as follows:

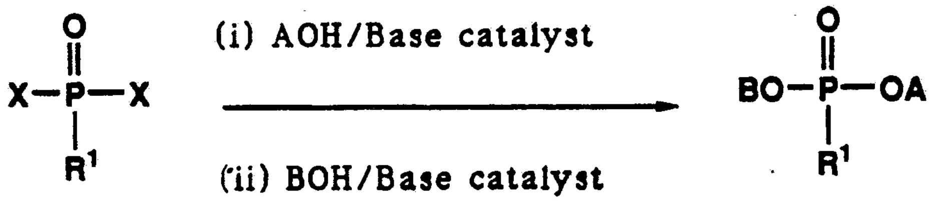


(XI) (I)

wherein A and B are as defined hereinbefore, X is halo, preferably chloro and $R^1$ is alkyl, hydroxyalkyl, alkoxyalkyl or acyl.

In step (i), compound (XI) can be contacted with compound AOH in the presence of catalytic amounts of a base catalyst in amounts and under conditions effective to displace -X with AOH. Generally, equimolar or excess amounts of compound (XI) to AOH can be employed at temperatures and solvents similar to those employed in steps (a) and (b) above.

The base catalyst can be a nitrogen-containing compound such as pyridine, N-methylimidazole, imidazole, or triazole, a tertiary amine such as triethylamine ($(CH_2CH_2)_3N$), or t-butylmagnesium chloride. In step (ii), the resultant compound from step (i) can be contacted with compound BOH in the presence of a base catalyst and under conditions similar to those employed in step (i) above.

One of ordinary skill in the art will recognize that steps (i) and (ii) can be conducted in reverse order by carrying out step (ii) prior to step (i) and still achieve similar results.

In a second alternative process for preparing the phosphonate esters of formula (I), the compound of formula (XI) wherein X is chloro can be contacted with either AOH or BOH in the presence of pyridine followed by contacting with 4-nitrophenol in the presence of pyridine, followed by further contacting with compound BOH or AOH in the presence of a base catalyst under conditions similar to those for step (i), above.

The sulfur derivatives of formula (I) wherein J is S can be readily prepared from the corresponding phosphate esters or phosphonate esters of formula (I) in the presence of a sulfurating agent. The preparation of the sulfur derivatives (I) is illustrated as follows:

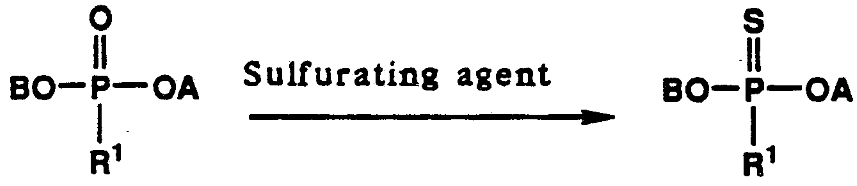


(I) (I)

Such methods for sulfurating can be found in J. March (ed.). Advanced Organic Chemistry, Reactions, Mechanisms and Structure, 3rd Edition, John Wiley & Sons, New York, N.Y. pp. 793-795, whose preparative teachings are incorporated herein by reference.

Example 1 illustrates a representative method for preparing a compound of the present invention but as such, should not be construed a limitations upon the overall scope of the same.

## EXAMPLE 1

5′-O-[[[3-(6-Amino-9H-purin-9-yl)cyclopentyl]methoxy]hydroxyphosphinyl]-3′-azido-3′-deoxy-thymidine

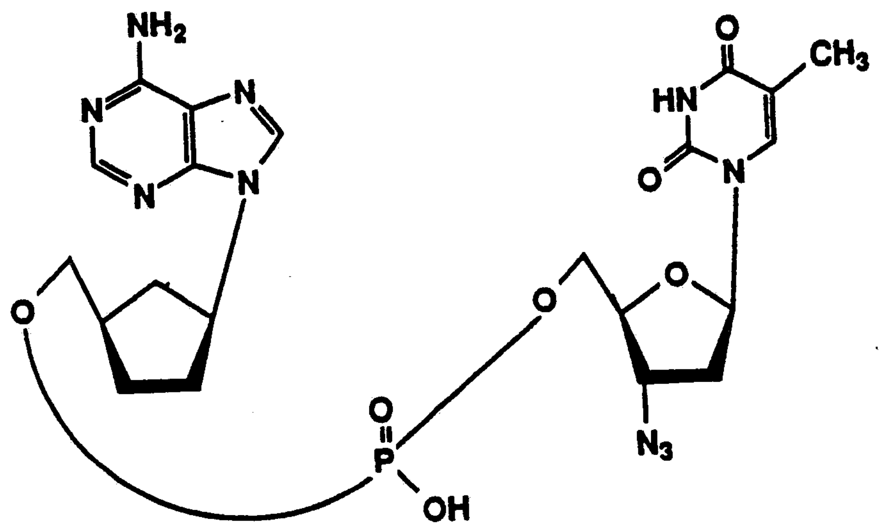

Azido-β-thymidine cyanoethyl phosphate (0.129g, 0.00032 mol) and 2′,3′-deoxyaristeromycin (0.075g, 0.00032 mol) are dissolved in 12 ml pyridine. To this solution is added 0.189g (0.00032x2.7 moles) of 2,4,6-trimethylphenylsulfonyl chloride. After stirring for about 30 minutes, 0.22 ml (0.00032x8 moles) N-methylimidazole is added and the mixture is stirred for about 24 hours in a stoppered flask. The reaction mixture is concentrated in vacuo and treated with 15 ml of 15 percent ammonium hydroxide. The resulting suspension is stirred at room temperature for about 24 hours and the insoluble solid is removed by filtration and washed with minimum water. The filtrate is concentrated in vacuo at a temperature of about 40° C and the last traces of water are removed by freeze drying. The crude product obtained is chromatographed on four thick plates using $CH_2Cl_2$:$CH_3OH$:$NH_4OH$ (30:10:1 volume basis) as eluent. The pure title compound is obtained as an amorphous solid by extraction of the middle band with methanol.

PMR ($CD_3OD$):1.84(m), 1.9(s), 1.93(m), 2.12(m), 2.28(m), 2.35(m), 2.45(m), 3.95(m), 4.1(m), 4.48(m), 4.9(m), 6.2(t), 7.75(s), 8.2(s), 8.29(s)

Mass Spec: FAB $[M+H]^+$ 563

PREPARATION OF STARTING MATERIALS

In one alternative procedure, the compound of formula (X) used either as an anti-viral agent or as an intermediation starting material to prepare the phosphate esters of formula (I) can be prepared by one or more of the following steps:

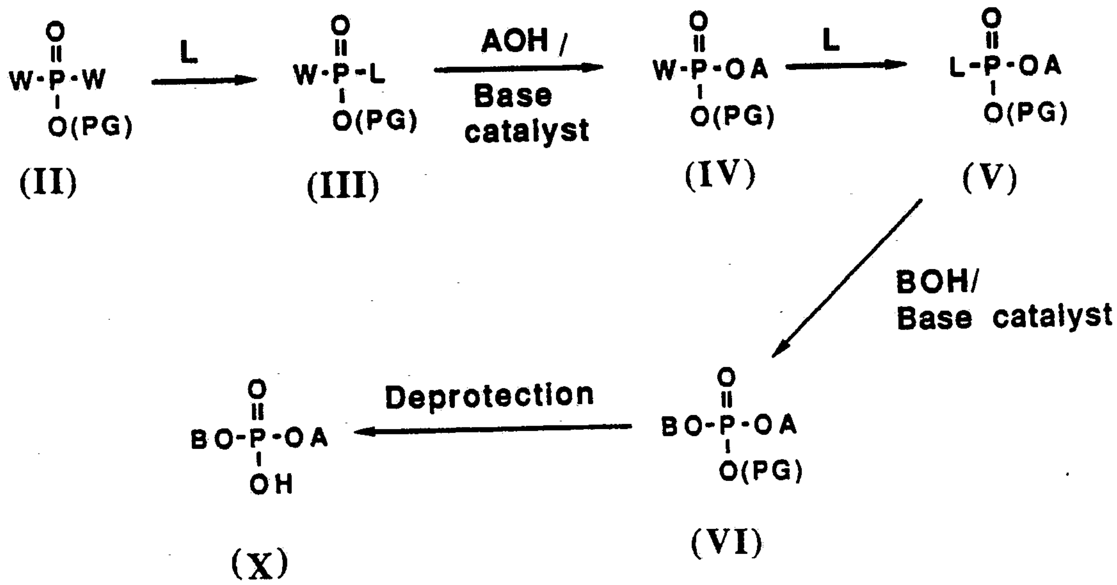


(II) (III) (IV) (V) (VI) (X)

wherein A and B are as defined hereinbefore;
W is chloro, iodo, or OH;
L is a leaving group which can be the same or different for each step, such as those described hereinbefore;
PG is a protecting group which can prevent reaction with AOH or BOH such as:
cyanoalkyl of 1 to 6 carbon atoms in the alkyl portion, preferably $NCCH_2CH_2$-;
haloalkyl of 1 to 6 carbon atoms, preferably $-CH_2CCl_3$ or $-CH_2CH_2CHBr_2$; or
halophenyl, preferably 2-chlorophenyl. The protecting group can subsequently be removed by conventional techniques to regenerate a hydroxy group.

One of ordinary skill in the art will recognize that the order of steps for preparing compound (X) can vary. For example, the BOH/base catalyst could be employed prior to utilizing AOH/base catalyst with similar results.

A general scheme for protection of the hydroxy is set further in (K.K. Ogilvie et al., Can. J. Chem. 56, pg. 2768 (1978); ibid. 57, pg. 2230 (1978), whose preparative teachings are incorporated herein by reference.

In a series of steps for preparing the requisite starting materials of formula (III), a compound of formula (II) can be contacted with a compound which can form a leaving group, L, in amounts and under conditions effective to allow the leaving group to replace the chloride, iodide or hydroxyl group of compound (II). Generally, approximately equimolar amounts of the compound forming the leaving group, to compound (II) can be employed, although excess amounts of the compound forming the leaving group can be used. The contacting can be carried out at any suitable temperature, preferably in the range from 0 to about 30 °C, preferably about 10 to 25 °C.

The reactants can be contacted at ambient pressures although pressures less than or greater than ambient can be employed. The reactants can be stirred or not stirred during the contacting, although stirring is preferred. The reactants are contacted for a time effective to complete the reaction, for a period ranging from about 5 minutes to about 24 hours or more. The contacting can be conducted in the presence of compatible solvents such as the chlorinated hydrocarbons including carbon tetrachloride ($CCl_4$), methylene chloride ($CH_2Cl_2$), and dichloroethane; aliphatics such as C-1 to C-20 alkanes, cyclic or acyclic; aromatics such as benzene, toluene, xylene, alkylbenzenes and the like; ethers such as diethyl ether and tetrahydrofuran (THF) and tertiary butyl-methyl ether; and solvents such as dimethylformamide (DMF), dimethylsulfoxide (DMSO), or mixtures thereof.

After the reaction is completed, the desired intermediate compound of formula (III) can be recovered by conventional separatory and recovery methods such as chromatography, distillation, crystallization and the like. Alternatively, compound (III) can be used directly to prepare other subsequent intermediates or starting materials.

The intermediate compound of formula (IV) can be prepared by contacting the compound of formula (III) with compound AOH in the presence of a base catalyst in amounts and under conditions effective to replace the leaving group, L, with -OA. Generally, approximately equimolar amounts HOA to compound (III) are employed, although excess amounts of AOH can be employed. The contacting can be carried out at any suitable temperature preferably in the range from 0 to about 30° C, preferably about 10 to 25° C.

The reactants can be contacted at ambient pressures although pressures less than or greater than ambient can be employed. The reactants can be stirred or not stirred during the contacting, although stirring is preferred. The reactants are contacted for a time effective to complete the reaction, for a period ranging from about 5 minutes to about 24 hours or more. The contacting can be conducted in the presence of compatible solvents such as the chlorinated hydrocarbons including carbon tetrachloride ($CCl_4$), methylene chloride ($CH_2Cl_2$), and dichloroethane; aliphatics such as C-1 to C-20 alkanes, cyclic or acyclic; aromatics such as benzene, toluene, xylene, alkylbenzenes and the like; ether such as diethyl ether and tetrahydrofuran (THF) and tertiary butyl-methyl ether; and solvents such as dimethylformamide (DMF), dimethylsulfoxide (DMSO), or mixtures thereof.

After the reaction is completed, the intermediate compound of formula (IV) can be recovered by separatory and recovery methods such as those described hereinbefore. Alternatively, compound (IV) can be used directly to prepare other subsequent intermediates.

The compound of formula (V) can be prepared according to procedures similar to those described for the preparation of compound (III).

The compound of formula (VI) can be prepared according to the procedures similar to those described for the preparation of compound (IV), except that compound BOH is used as a reactant.

The compound of formula (X) can be prepared by deprotecting the protecting group, PG, of compound (VI) to the hydroxy group of formula (X). Generally, the compound of formula (VI) can be treated according to the deprotecting procedures described in F. Ramirez and J. F. Marecek, Synthesis of Phosphodiesters: The Cyclic Enediol phosphoryl (CEP Method), Synthesis, May 1985, pp. 449-488, whose preparative teachings are incorporated herein by reference. One of ordinary skill in the art will recognize that the phosphate ester compounds of Formula (X) constitutes a subgroup of Formula (I) in which $R^1$ is OH. If desired, the compounds (X) can be employed as intermediates for preparing compounds (I) wherein $R^1$ is other than OH.

The compounds of formula AOH and BOH can be prepared by the processes similar to those described in European Patent Application Publication No. 0 338 842 published October 25, 1989. The compounds may also be prepared by the processes described in European Patent Application 236,935. The disclosures of the above mentioned applications are incorporated herein by reference herein for their preparative teachings.

The starting materials of formula (II) are known and can be prepared by known procedures, such as those described in Marquez and Ma-III Lim supra, whose preparative teachings are incorporated herein by reference.

Example 2 which follows illustrates a representative method for preparing the starting materials used to prepare the compounds of the present inventions but as such should not be construed as limitations upon the overall scope of the same.

## EXAMPLE 2

## PREPARATIVE EXAMPLE

Step (a) : Preparation of 2′,3′-Cyclic Ortho Esters

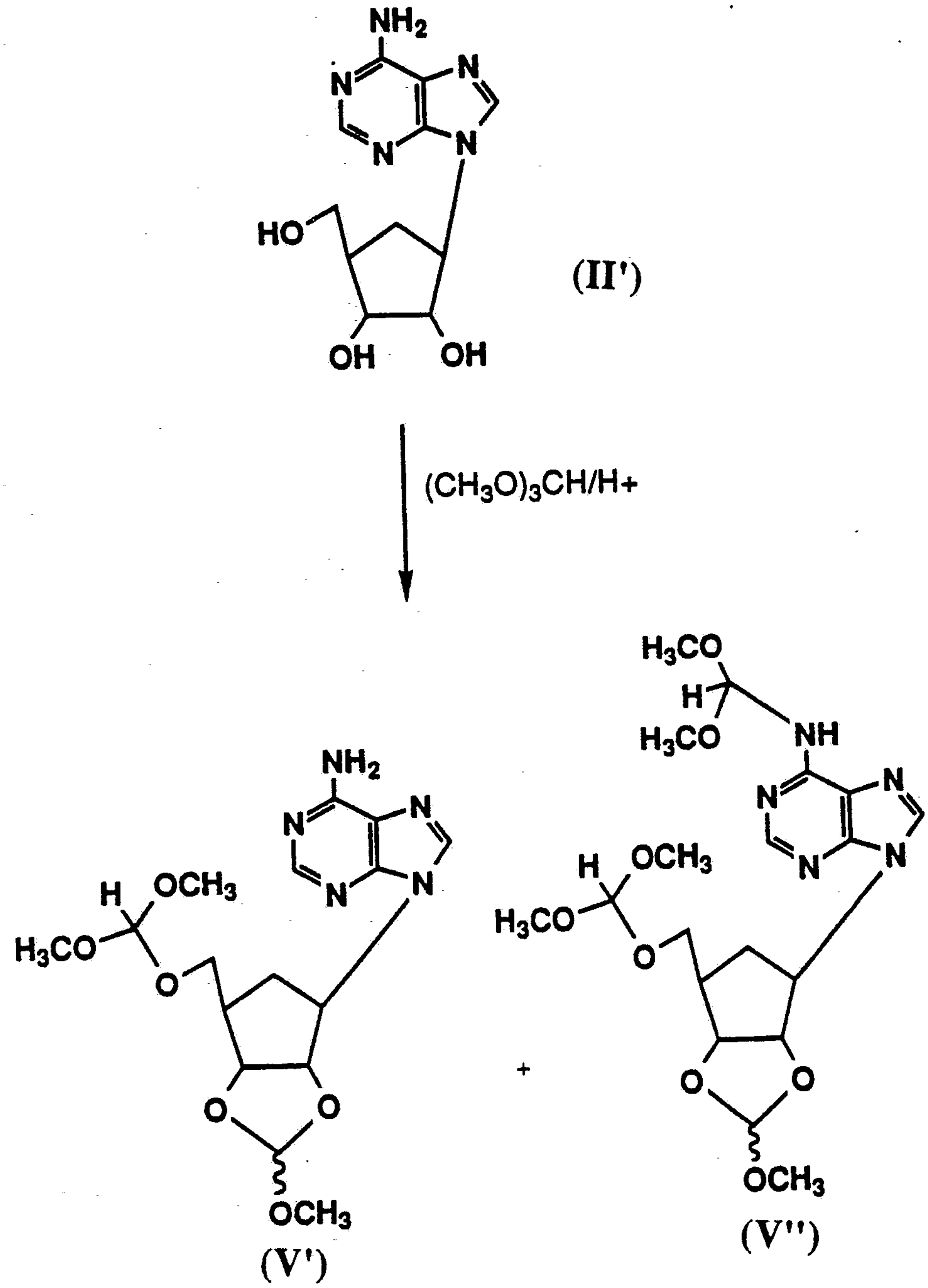


(II')

(V') + (V'')

Aristeromycin 10 grams (g) is stirred with 100 milliliters (ml) methyl orthoformate ($(CH_3O)_3CH$) and dry dimethylformamide (30 ml) in the presence of p-toluenesulfonic acid monohydrate (10.3 g). After about 36 hours stirring at room temperature, 15 g anhydrous potassium carbonate ($K_2CO_3$) is added and the mixture is stirred for about 2 hours. The solids containing potassium tosylate are removed by filtration and washed with minimum amounts of methyl orthoformate and toluene. The combined filtrates and washings are evaporated in-vacuo in a bath having a temperature of 40° to 50°C to provide a gummy residue. The gummy residue is azeotroped with 100 ml toluene on a rotary evaporator to remove the methyl orthoformate, and gives a mixture of title 2′,3′-cyclic ortho esters (V′) and (V″), together with a small amount of unidentified compounds. Proton magnetic resonance spectroscopy (PMR) ($CDCl_3$):

δ 1.8(m), 2.47(m), 2.88(s), 2.95(s), 3.27(s), 3.29(s), 3.35(s), 3.42(s), 3.44(s), 3.74(m), 3.9(m), 4.8(m), 5.09(d), 5.15(m), 5.62(broad s), 5.65(m), 5.88(d), 5.94(s), 7.84(s), 7.85(s), 7.86(s), 8.32(s), 8.33(s).

These compounds are used in step (b) infra.

Step (b): Preparation of 2′,3′-Dehydro derivatives

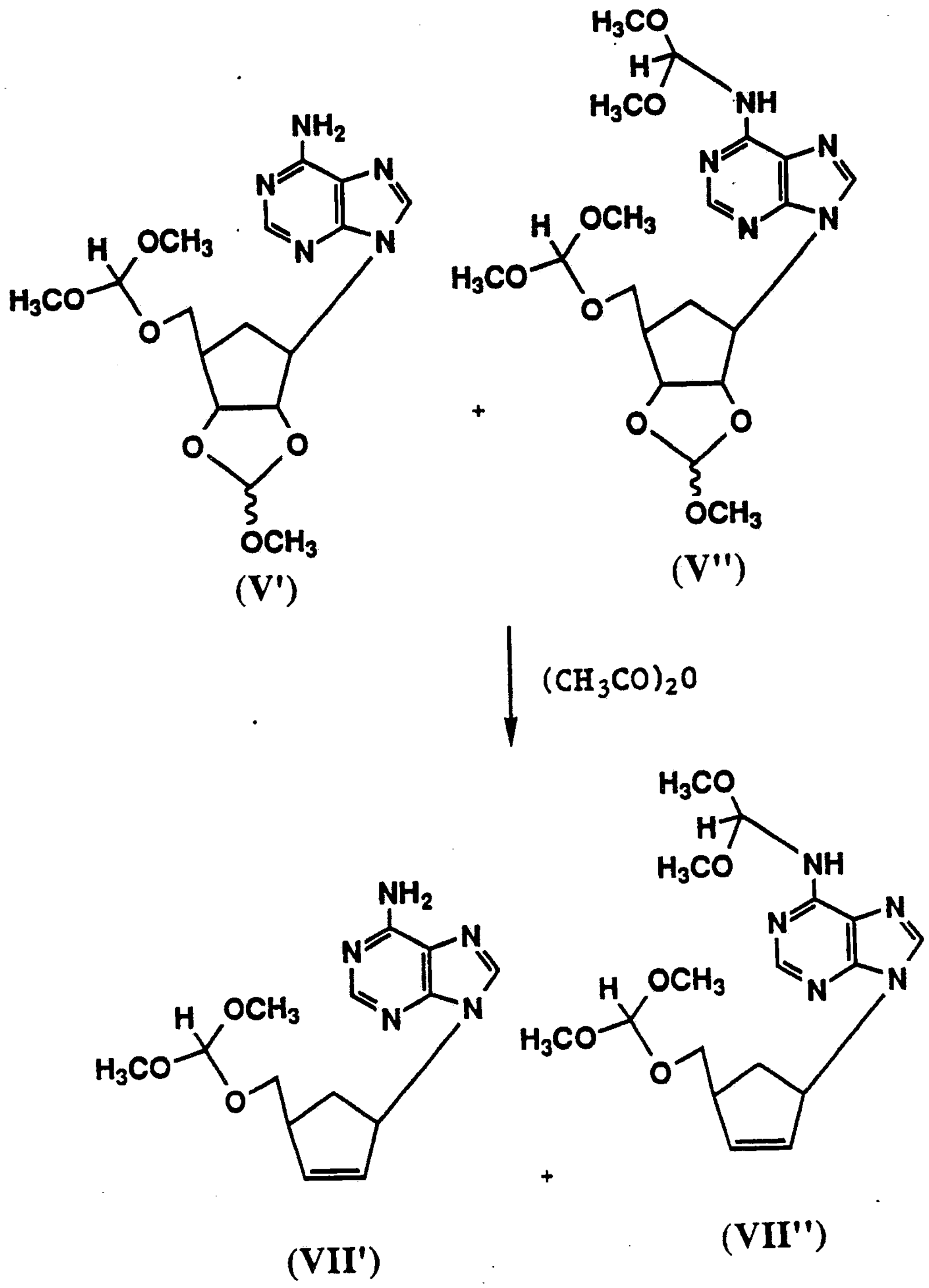


(V') + (V'')

(VII') + (VII'')

The mixture from step (a) is first refluxed overnight with 100 ml acetic anhydride ($(CH_3CO)_2O$) under argon atmosphere. After heating overnight, the dark reaction mixture is evaporated in-vacuo and the dark brown gummy product remaining is filtered through a column of silica ($SiO_2$) (300 g). The column is prepared in 2 percent methanol/methylene chloride. After a 500 ml forerun, the eluting fractions of 300 ml per fractions are collected. Fractions 2 through 8 are combined to give the 2′,3′-dehydro derivatives (VII′ and VII″), together with a small amount of unidentified compounds.

PMR ($CDCl_3$): δ 1.7(m), 1.85(s), 1.97(s), 2.01(s), 2.28(s), 2.54(s), 2.9(m), 3.18(m), 4.1(m), 5.0(m), 5.75-5.8(m), 5.9(m), 6.17(m), 8.09(s), 8.1(s), 8.16(s), 8.67(s), 8.89(s), 9.4-9.5(m).

These compounds are used in the step (d) without further purification. Alternatively, 2′,3′-dihydro derivatives (VII′ and VII″) can be deprotected according to deprotection procedures described herein, to provide starting materials for preparing the desired compounds of Formula (I).

Step (d): Preparation of Cyclopentyl Purine Derivatives

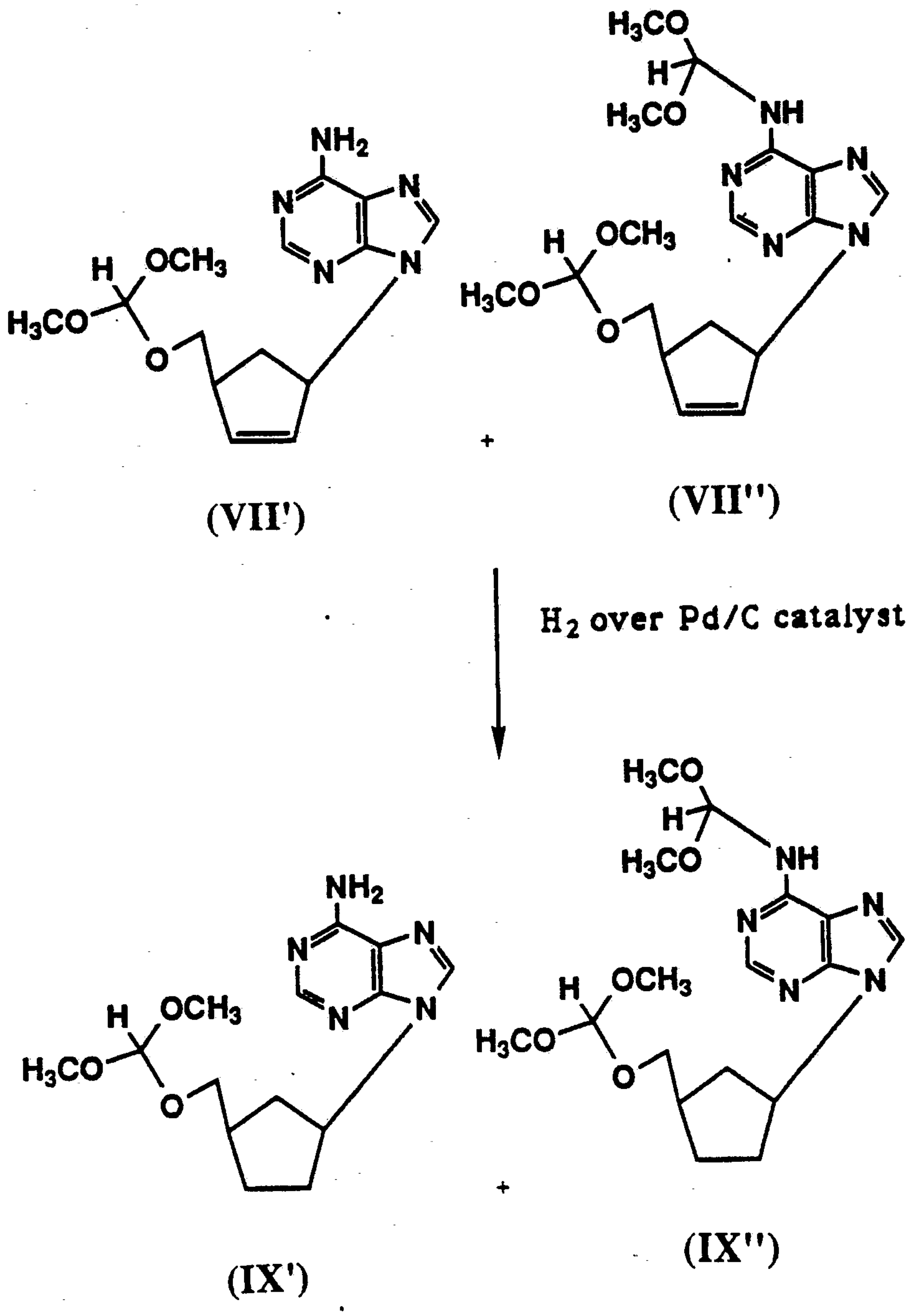


(VII') + (VII'')

(IX') + (IX'')

The 2′,3′-dehydro derivatives (VII′ and VII″) obtained in step (c) are dissolved in ethanol and hydrogenated with 5 percent Palladium/carbon (Pd/C) (0.15 g) in a Paar hydrogenator until no starting material can be detected by thin-layer chromatography. The catalyst is removed by filtration, the reaction mixture is washed with ethanol and the combined filtrate and washings are evaporated to dryness in vacuo to give 9.18 of the cyclopentyl purine derivatives (IX′ and IX″), together with a small amount of unidentified compounds.

Step (e): Preparation of 6′-Hydroxy cyclopentyl derivative (2′,3′-deoxyaristeromycin)

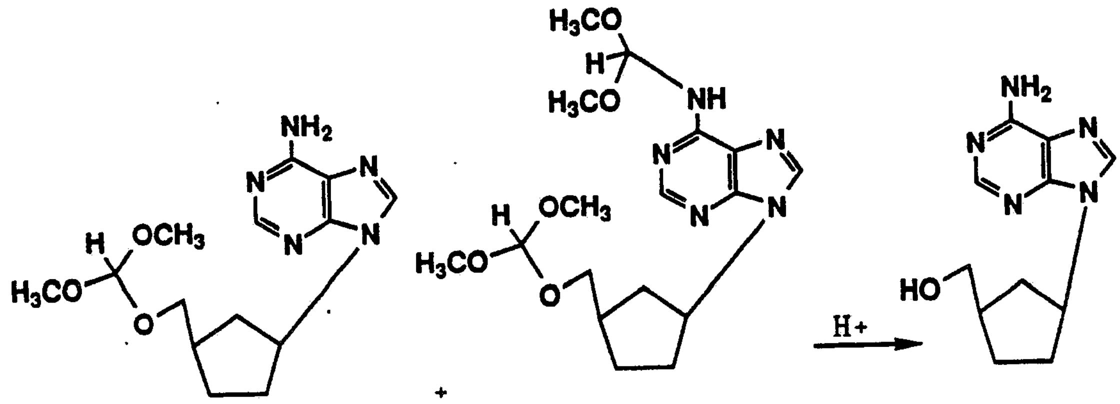


(IX') + (IX'') (X')

The cyclopentyl purine derivatives from step (c) are dissolved in 6 Normal (N) HCl (120 ml) and let to stand overnight at room temperature. A small amount of gummy precipitate present is removed by filtration. The aqueous acidic solution is concentrated to dryness in-vacuo, and the residue is taken up in 60 ml concentrated $NH_4OH$ (28 percent). After four hours the aqueous solution is concentrated and the residue remaining is chromatographed on silica gel (160 g). The column is eluted with 5 to 8 percent methanol/methylene chloride and 150 ml fractions are collected. Fractions containing the desired 6,-hydroxy cyclopentyl derivative, as determined by thin layer chromatography, are combined to provide 4.08 g of 2′,3′-deoxyaristeromycin (X′), a solid. A small portion of the solid is recrystallized from ethyl acetate to give rod-shaped crystals, melting point 127-128° C.

PMR (DMSO-$d_6$): 1.65(m), 1.75(m), 2.05(m), 2.15(m), 2.25(m), 2.5 (broad s), 3.42(m), 4.57(t), 4.8(m), 7.12(s), 8.1(s), 8.2(s).

Mass Spec.: FAB $[M+H]^+$ 234

$[\alpha]_D^{26}$ - 6.3° (9.83 mg/2ml $H_2O$)

Analysis: $C_{11}H_{15}ON_5$ (Molecular weight: 233)

Calculated - C:56.63, H:6.48, N:30.02

Found - C:56.60, H:6.48, N:30.68

The structure of 2′,3′-Deoxyaristeromycin is further confirmed by X-ray analysis.

The compounds of formula (I) can be produced by the following processes A and B as follows:

A. To form a compound of Formula (I) wherein $R^1$ is alkoxy, alkoxyalkoxy, phenoxy or an OB moiety, react a compound of Formula (Xa)

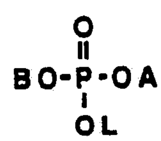

(Xa)

herein A and B are as defined hereinbefore and L is a leaving group defined hereinbefore, with an alcohol of the Formula ROH (XX) wherein RO is the same as $R^1$ when $R^1$ is alkoxy, alkoxyalkoxy, phenoxy, substituted phenoxy or an OB moiety, to yield the phosphate ester of the Formula (I)

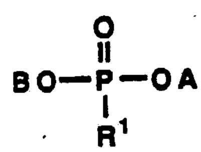

(I)

wherein B and A are as defined in Process A and

$R^1$ represents alkoxy, alkoxyalkoxy, phenoxy, or an OB moiety in compound (I); and

B. To form a compound of Formula (I) wherein $R^1$ is alkyl, hydroxyalkyl, alkoxyalkyl or acyl, react a compound of Formula (XI)

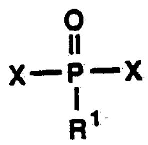

(XI)

with a compound of the Formula AOH together with a base catalyst followed by, or at the same time, reaction with a compound of Formula BOH together with a base catalyst, or alternatively, wherein BOH is reacted with the compound of Formula (XI) before reaction with AOH,
wherein A and B are as defined previously, X is chloro and $R^1$ is alkyl, hydroxyalkyl, alkoxyalkyl or acyl to yield a compound of Formula (I)

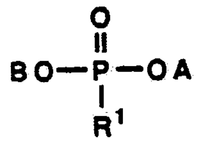

(I)

wherein B, A and $R^1$ are as defined for Process B, and
if desired converting a compound of formula I to a different compound of formula I by conventional means,
if necessary or desired removing any protecting group; and
if desired, forming a solvate or pharmaceutically acceptable salt of the compound so produced.

The compounds of the present invention are active against viruses, such as HIV. The present compounds can be pharmaceutical compositions which can be administered parenterally or orally. Preferably, the compounds are administered orally. The compounds can be administered in a dosage range of about 0.000001 to 0.01 grams per kilogram ( g/kg) body weight per day. It is believed that the compounds of this invention are non-toxic when administered in this dosage range.

Pharmaceutical compositions comprising the compounds of this invention may be prepared from standard ingredients using standard techniques. For example, injectable formulations use aqueous physiologically acceptable carriers, such as distilled water.

Oral compositions include tablets, capsules, syrups, elixirs and suspensions. The typical acceptable pharmaceutical carriers for use in the oral formulations described above are exemplified by: sugars such as lactose, surcrose, mannitol and sorbitol; starches such as corn starch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone, polyvinyl alcohol; stearic acid; alkaline earth metal stearates such as magnesium stearate and calcium stearate, stearic acid, vegetable oils such as peanut oil, cottonseed oil, sesame oil, olive oil and corn oil; non-ionic, cationic and anionic surfactants; ethylene glycol polymers; beta-cyclodextrin; fatty alcohols and hydrolyzed cereal solids; as well as other non-toxic compatible filters, binders, disintegrants, buffers, antioxidants, lubricants, flavoring agents, and the like commonly used in pharmaceutical formulations.

In addition to the pharmaceutical carrier and active drugs, the contemplated formulations can also contain preservatives, such as glycerine or stabilizers.

Compositions of the present invention can be administered one or more times daily, preferably 1 to 3 times daily. The exact dosage to be administered is based upon the judgment of the clinician, and is dependent upon such factors as the weight and condition of the individual. Examples of compositions follow wherein 5′-O-[[[3-(6-amino-9H-purin-9-yl)cyclopentyl]methoxy]hydroxyphosphinyl]-3′-azido-3′-deoxythymidine is the active ingredient of the invention:

| | Concentration | |
|---|---|---|
| Capsules: | mg/capsule | mg/capsule |
| Active Ingredient | 50 mg | 150 mg |
| Lactose USP | 106 | 73 |
| Corn Starch, Food Grade | 40 | 70 |
| Magnesium Stearate | 4 | 2 |

Blend the ingredients and fill into hard gelatine capsules.

| | Concentration |
|---|---|
| Oral liquid: | mg/capsule |
| Active Ingredient | 50 |
| Sucrose | 800 |
| Glycerin | 0.4 |
| Water | q.s. 1 ml. |

The following example is intended to illustrate, but not to limit, the present invention.

## EXAMPLE 3

Continuous cell lines derived from human lymphocytes are grown in a microtiter test system. The cells are infected with strain HIV-III of the Human Immunodeficiency virus (HIV), a standard strain isolated from a patient with Acquired Immune Deficiency Syndrome (AIDS). The treated cells are incubated for 7 to 14 days, and the treated cells are inspected by microscope to determine (a) prevention of synctia (cell to cell fusion) formation induced by HIV, or (b) prevention of cell rounding and prevention of HIV caused cytopathology. Such inspections indicate the ability of the compound to prevent cell killing by the virus. Additionally, biochemical determination of the cell media is made to ascertain the ability of the compound to prevent formation of viral products (reverse transcriptase, p24 antigen, or other products), and prevention of formation of more HIV itself. A compound is determined to be active if it exhibits any of the evaluation criteria described above.

Table 1 shows the activity of a selected compound of the present invention to protect cells from virus destruction by HIV as indicated in tests (a) and (b) described hereinbefore. The control refers to the treatment without a compound of the present invention.

Table 1.

| Protection of cells from Virus Destruction by HIV | | |
|---|---|---|
| Compound | Concentration | Protection of Cells |
| | (µg/ml) | ---percent--- |
| Compound of Example 1 | 32<br>100 | 100<br>100 |
| Control | -- | 0 |

While the present invention has been described in conjunction with the specific embodiments set forth above, many alternatives, modifications and variations thereof will be apparent to those of ordinary skill in the art. All such alternatives, modifications and variations are intended to fall within the spirit and scope of the present invention.

## Claims

1. A compound of the formula (I)

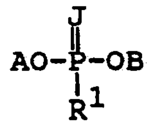 (I)

or a pharmaceutically acceptable salt or solvate thereof, wherein:
J is an oxygen or sulfur atom;
A represents

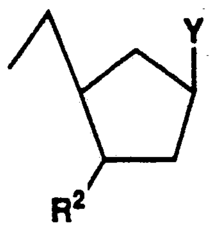 , 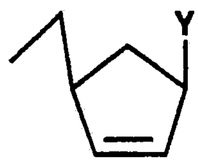 , 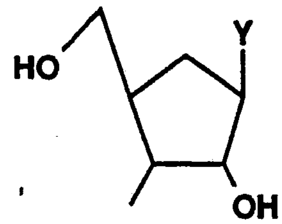 or 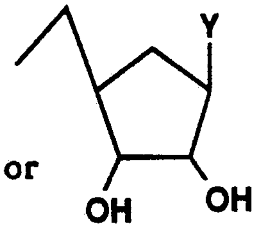 ;

B represents

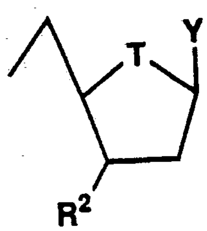 , 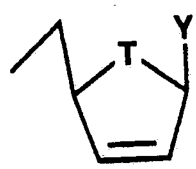 , 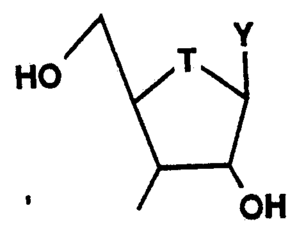 or 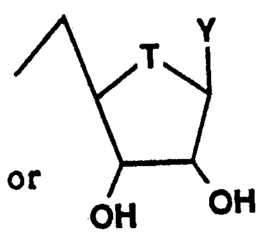 ;

$R^1$ represents hydroxy, alkyl, alkoxy, hydroxyalkyl, hydroxyalkoxy, alkoxyalkyl, alkoxyalkoxy, acyl, phenoxy, substituted phenoxy, or OB where B is defined as above and each B may be the same or different;
each Y independently represents

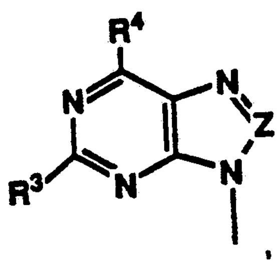 , 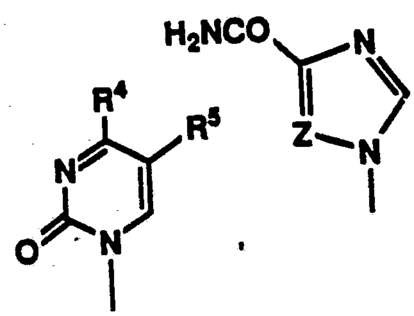 , or 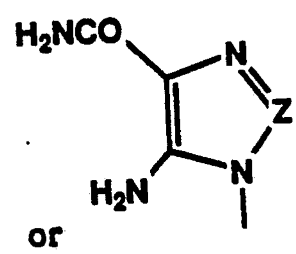 ;

each $R^2$ independently represents H, F, (F,F), OH, $NO_2$, azido, alkoxy, amino, alkylamino, dialkylamino or amide;
each T independently represents O or $CH_2$;
$R^3$ and $R^4$ can be the same or different and each is independently selected from H, halo, OH, SH, alkoxy, alkylthio, amino, alkylamino, dialkylamino or amide;
each $R^5$ independently represents H, halo, $-CF_3$, vinyl, halovinyl, alkyl, hydroxyalkyl, alkoxyalkyl, -COOH, -COOalkyl or acyl; and
each Z independently represents N or CH.

2. A compound according to claim 1, wherein J is O, $R^1$ is OH and B represents

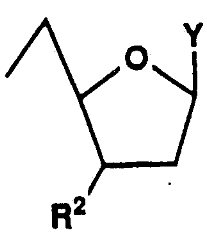,
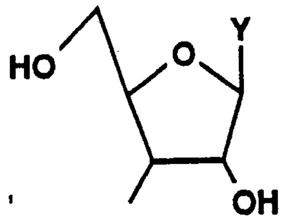
or
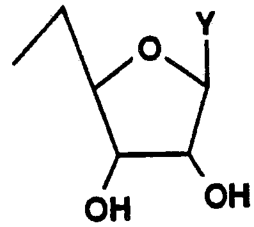
;

wherein $R^2$ and Y are as defined in claim 1.

3. A compound according to claim 1, wherein J is O, $R^1$ is OH and B represents the same values as for A as defined in claim 1.

4. A compound according to claim 2, wherein each Y is selected from

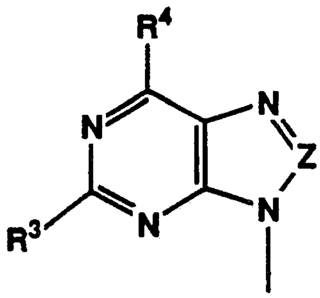
,
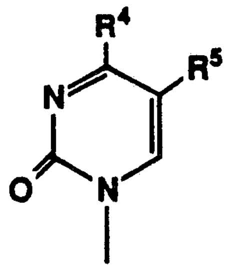
or
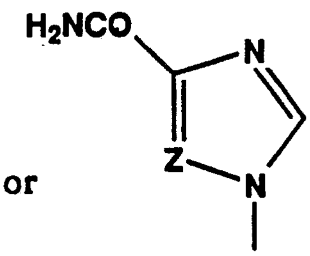
;

wherein $R^3$, $R^4$ and Z are defined in claim 1; and
each $R^5$ represents halo, alkyl, $-CF_3$, vinyl, halovinyl, $-CH_2OH$, $-CH_2Oalkyl$, -COOH or -COOalkyl.

5. A compound according to claim 3, wherein Y in group A represents

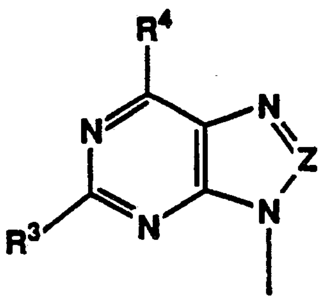
,
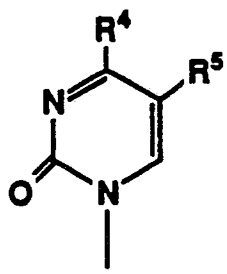
or
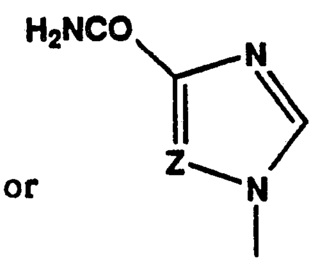
;

wherein $R^3$, $R^4$ and Z are as defined in claim 1; and
each $R^5$ represents halo, alkyl, $-CF_3$, vinyl, halovinyl, $-CH_2OH$, $-CH_2Oalkyl$, -COOH or -COOalkyl.

6. A compound according to claim 1, wherein the compound is one of formulas Ia-In below:

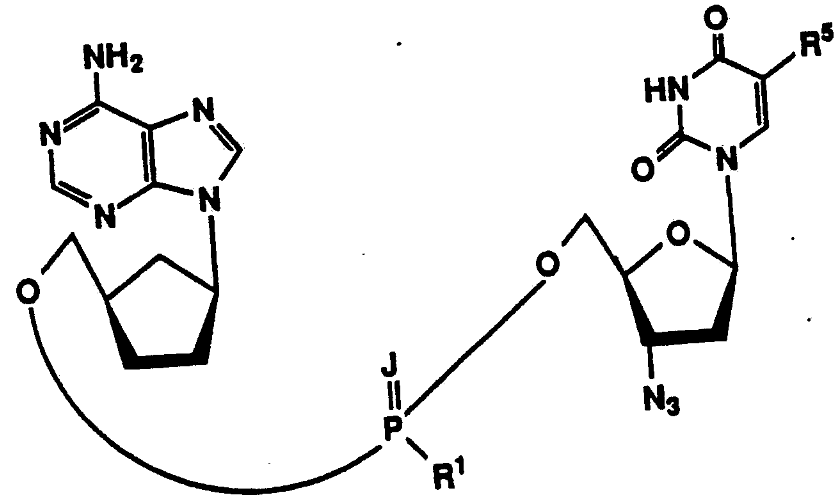

Ia

wherein J is O or S, $R^1$ is OH, $OCH_3$, $CH_3$ or OB where B is as defined in claim I, and $R^5$ is H, $CH_3$ or halo;

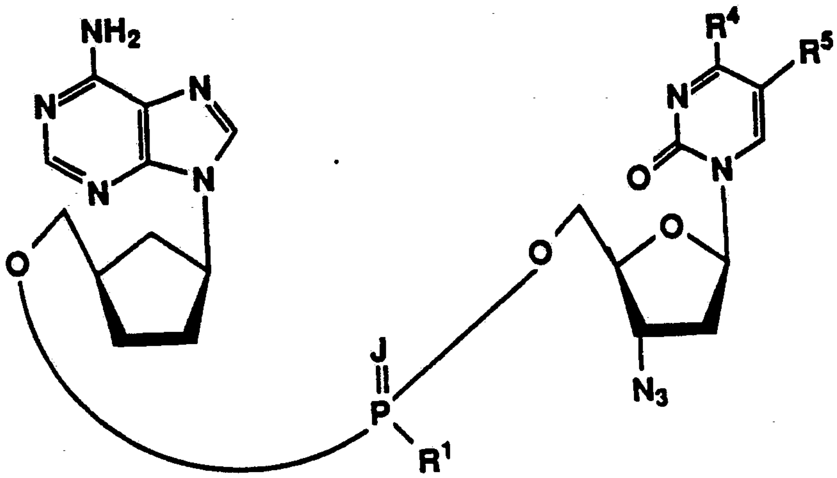

Ib

wherein J is O or S, $R^1$ is OH, $OCH_3$ or $CH_3$, $R^4$ is OH or $NH_2$, and $R^5$ is H, $CH_3$ or halo;

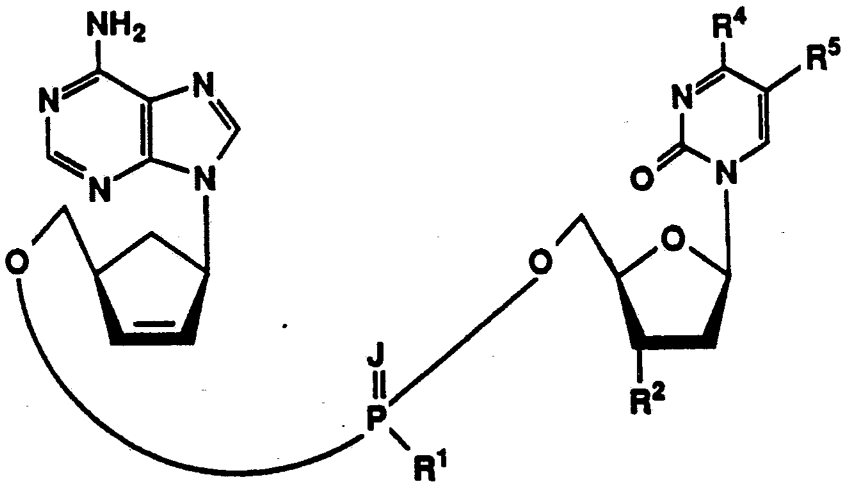

Ic

wherein J is O or S, $R^1$ is OH, $OCH_3$, $CH_3$ or OB where B is as defined in claim 1, $R^2$ is H or $N_3$, $R^4$ is OH or $NH_2$, and $R^5$ is H, $CH_3$ or halo;

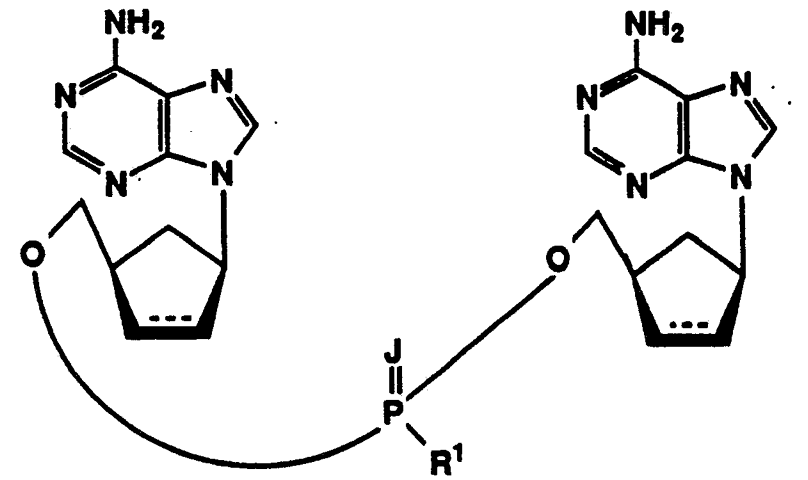

Id

wherein J is O or S, $R^1$ is OH, $OCH_3$, $CH_3$ or OB where B is as defined in claim 1 and the dotted lines indicate optional double bonds;

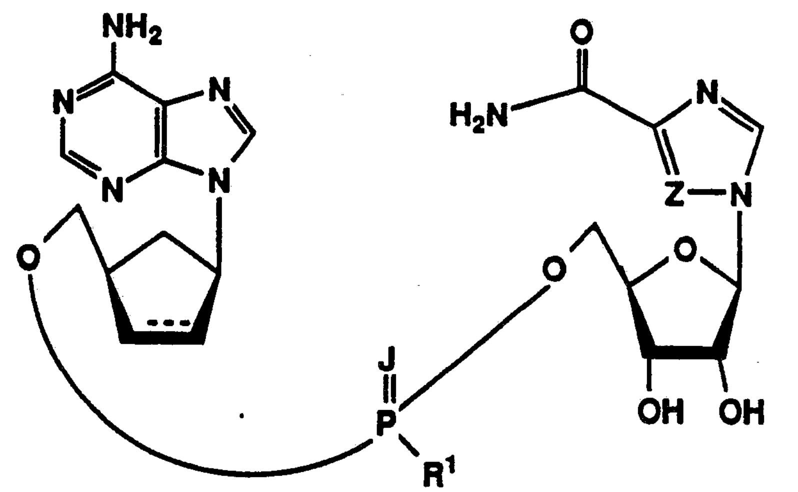

Ie

wherein J is O or S, $R^1$ is OH, $OCH_3$, $CH_3$ or OB where B is as defined in claim 1, Z is N or CH, and the dotted line indicates an optional double bond;

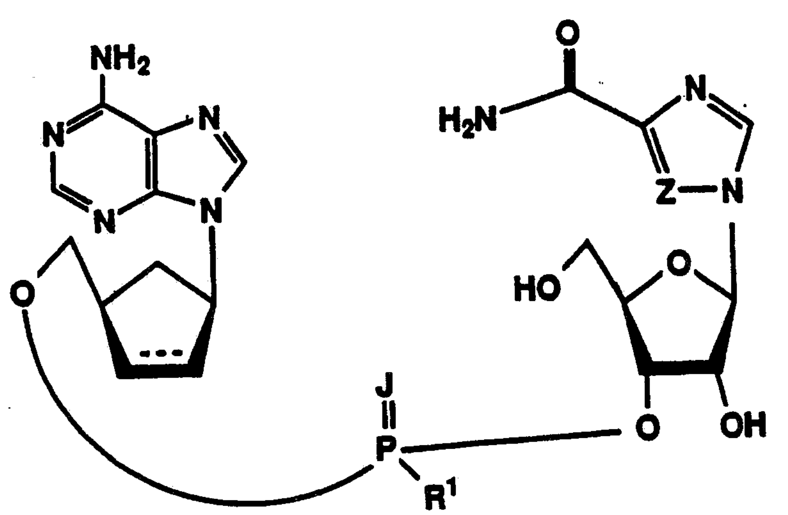

If

wherein J is O or S, $R^1$ is OH, $OCH_3$, $CH_3$ or OB where B is as defined in claim 1, Z is N or CH, and the dotted line indicates an optional double bond;

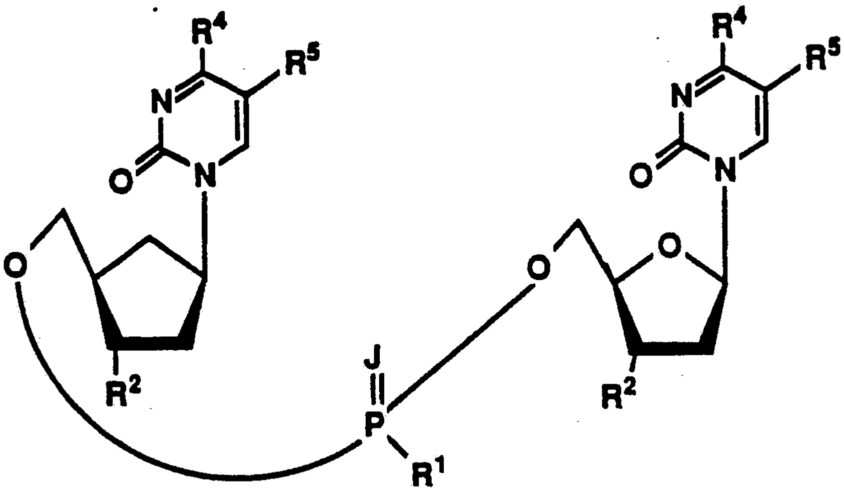

Ig

wherein J is O or S, $R^1$ is OH, $OCH_3$, $CH_3$ or OB where B is as defined in claim 1, $R^2$ is H or $N_3$, each $R^4$ is independently OH or $NH_2$, and each $R^5$ is independently H, $CH_3$ or halo;

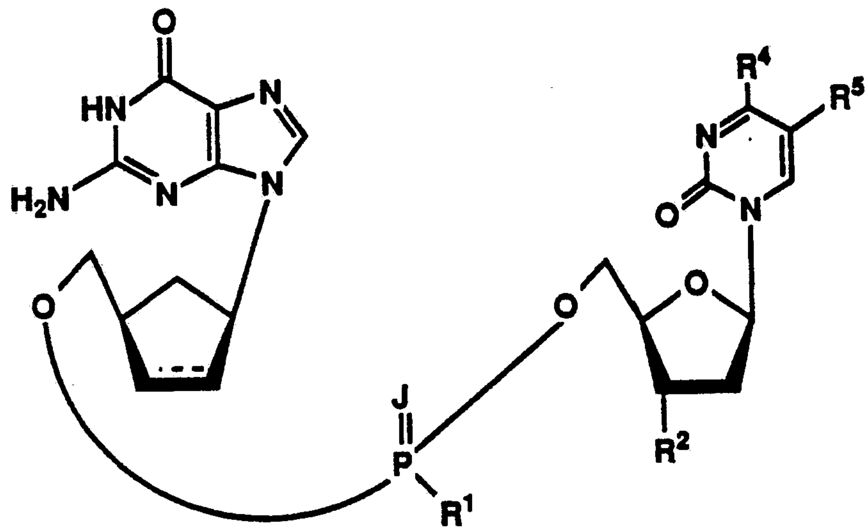

Ih

wherein J is O or S, $R^1$ is OH, $OCH_3$, $CH_3$ or OB where B is as defined in claim 1, $R^2$ is H or $N_3$, $R^4$ is OH or $NH_2$, $R^5$ is H, $CH_3$ or halo, and the dotted line indicates an optional double bond;

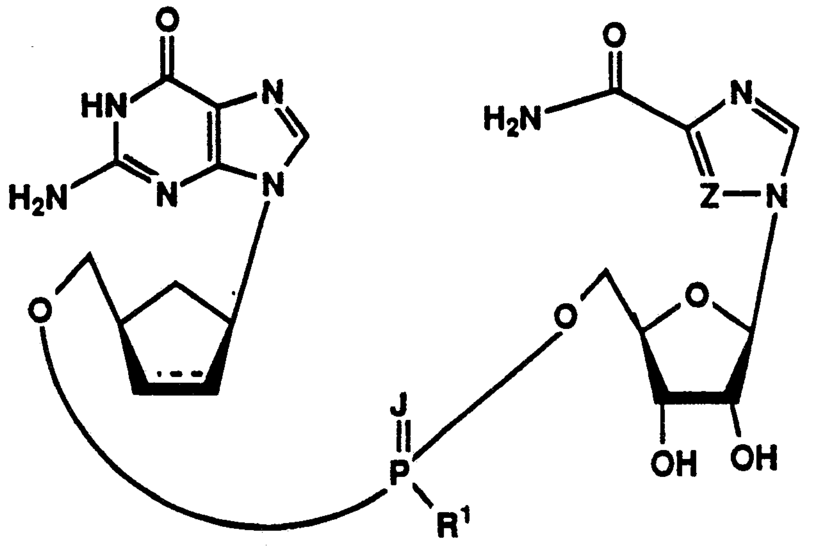

Ii

wherein J is O or S, $R^1$ is OH, $OCH_3$, $CH_3$ or OB where B is as defined in claim 1, Z is N or CH, and the dotted line indicates an optional double bond;

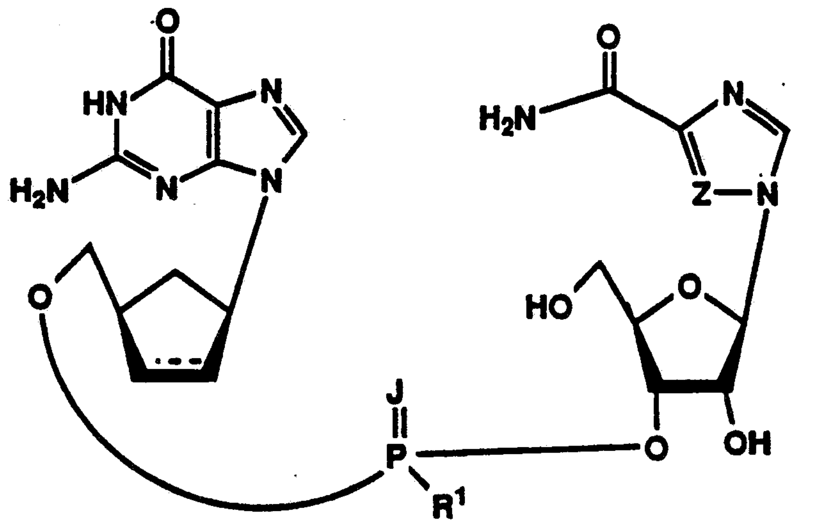

Ij

wherein J is O or S, $R^1$ is OH, $OCH_3$, $CH_3$ or OB where B is as defined in claim 1, Z is N or CH, and the dotted line indicates an optional double bond;

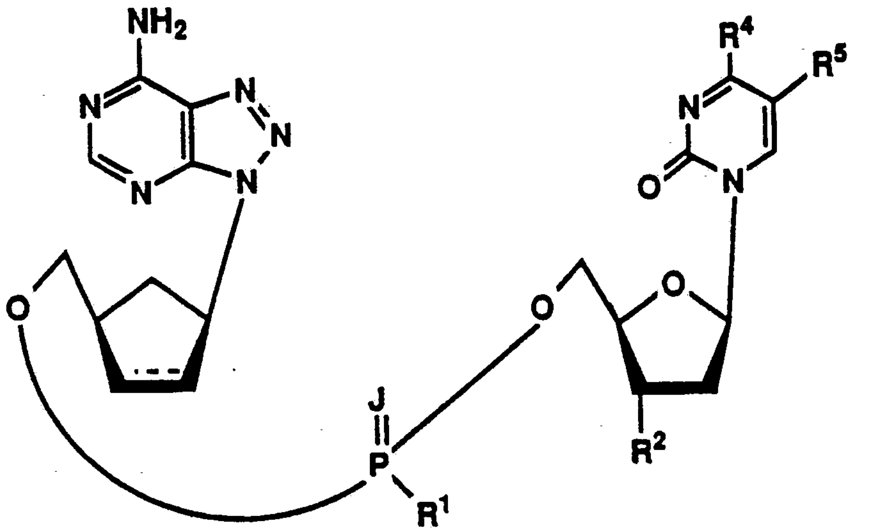

Ik

wherein J is O or S, $R^1$ is OH, $OCH_3$, $CH_3$ or OB where B is as defined in claim 1, $R^2$ is H or $N_3$, $R^4$ is OH or $NH_2$, $R^5$ is H, $CH_3$ or halo, and the dotted line indicates an optional double bond;

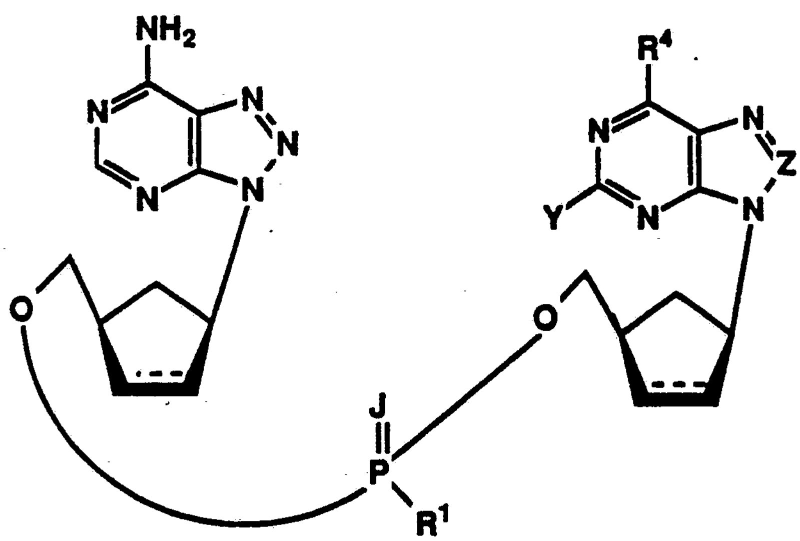

Il

wherein J is O or S, $R^1$ is OH, $OCH_3$, $CH_3$ or OB where B is as defined in claim 1, $R^3$ is H or $NH_2$, $R^4$ is OH or $NH_2$, Z is N or CH, and the dotted lines indicate an optional double bond;

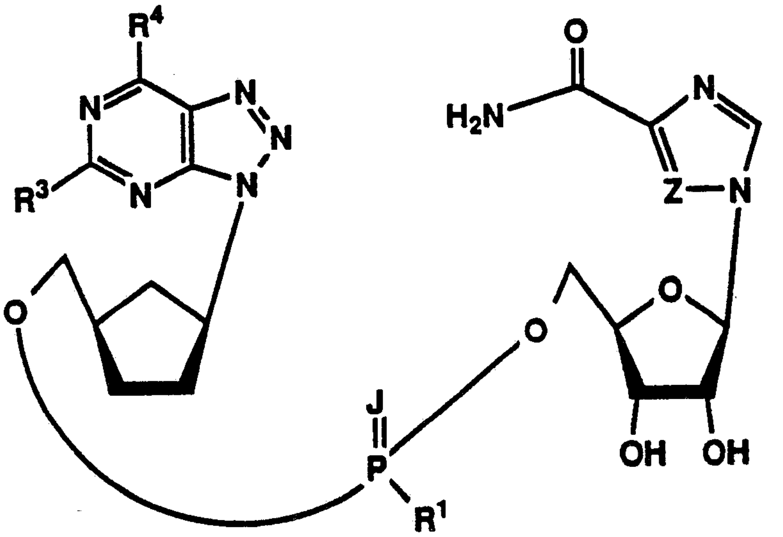

Im

wherein J is O or S, $R^1$ is OH, $OCH_3$, $CH_3$ or OB where B is as defined in claim 1, $R^3$ is H or $NH_2$, $R^4$ is OH or $NH_2$ and Z is N or CH; or

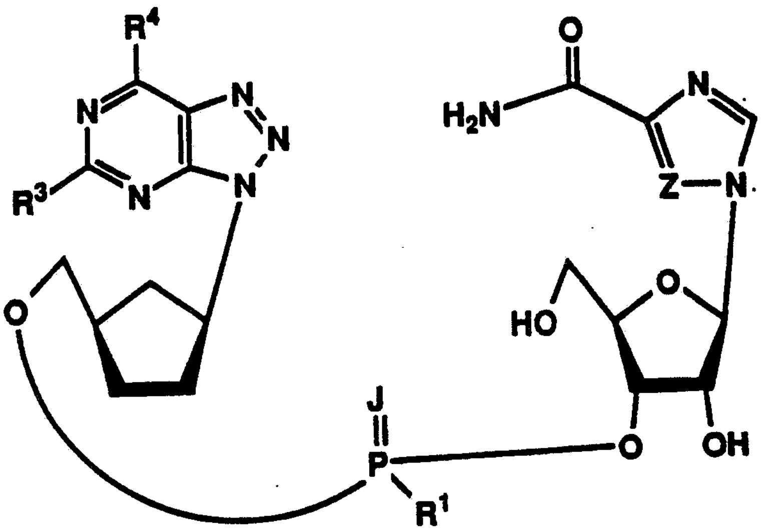

In

wherein J is O or S, $R^1$ is OH, $OCH_3$, $CH_3$ or OB where B is as defined in claim 1, $R^3$ is H or $NH_2$, $R^4$ is OH or $NH_2$, and Z is N or CH.

7. A compound according to claim 1, which is 5′-O-[[[3-(6-amino-9H-purin-9-yl)cyclopentyl]methoxy]hydroxyphosphinyl]-3′-azido-3′-deoxy-thymidine.

8. A pharmaceutical composition comprising a pharmaceuticaly effective amount of a compound of formula I as defined in any of claims 1 to 7 in combination with a pharmaceutically acceptable carrier.

9. A method of treating susceptible viral infections in a mammal comprising administering to said mammal an anti-viral effective amount of a compound of formula I as defined in any of claims 1 to 7.

10. The use of a compound of formula I as defined in any one of claims 1 to 7 for the manufacture of a medicament for use in treating susceptible viral infections in a mammal.

11. The use of a compound of formula I as defined in any one of claims 1 to 7 for the treatment of susceptible viral infections in a mammal.

European Patent Office

# PARTIAL EUROPEAN SEARCH REPORT

which under Rule 45 of the European Patent Convention shall be considered, for the purposes of subsequent proceedings, as the European search report

Application number
EP 89 31 2467

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF MEDICINAL CHEMISTRY, vol. 11, no. 1, January 1968, pages 44-48, Columbus, Ohio, US; H.J. THOMAS et al.: "Derivatives and analogs of 6-mercaptopurine ribonucleotide"<br>* Abstract; page 45, compound 13; page 45, table 1, compound 13 *<br>-- | 1,3,5, 8,10, 11 | C 07 H 19/04<br>A 61 K 31/70<br>C 07 H 19/10<br>C 07 H 19/20 |
| A | BIOCHEMISTRY, vol. 27, no. 1, 1988, pages 183-193, Columbus, Ohio, US; J.T. SLAMA et al.: "Carbanicotinamide adenine dinucleotide: Synthesis and enzymological properties of a carbocyclic analogue of oxidized nicotinamide adenine dinucleotide"<br>* Page 184, compound "Carba-NAD, 1" *<br>-- ./. | 1 | TECHNICAL FIELDS SEARCHED (Int. Cl.4)<br>C 07 H 19/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8,10

Claims searched incompletely:

Claims not searched: 9,11

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 02-04-1990 | SCOTT |

CATEGORY OF CITED DOCUMENTS

- X : particularly relevant if taken alone
- Y : particularly relevant if combined with another document of the same category
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document

European Patent Office

# PARTIAL EUROPEAN SEARCH REPORT

Application number
EP 89 31 2467
-2-

| DOCUMENTS CONSIDERED TO BE RELEVANT | | | CLASSIFICATION OF THE APPLICATION (Int Cl.4) |
|---|---|---|---|
| Category | Citation of document with indication, where appropriate, of relevant passages | Relevant to claim | |
| A | JOURNAL OF MEDICINAL CHEMISTRY, vol. 28, no. 9, September 1985, pages 1376-1380, The American Chemical Society, Columbus, Ohio, US; H. SAWAI et al.: "Replacement of the ribofuranose oxygen of 2-5A derivatives by methylene: Synthesis of an aristeromycin analogue of 2-5A core 5'-monophosphate (5'-O-phosphoryladenylyl)(2'→5')adenylyl(2'→5')adenosine" | | |
| | * Page 1376, scheme 1, compounds 8A,8B * | 1 | |
| | ---- | | TECHNICAL FIELDS SEARCHED (Int Cl.4) |